(19) Europäisches Patentamt
European Patent Office
Office européen des brevets

(11) Publication number: 0 359 617
A2

(12) # EUROPEAN PATENT APPLICATION

(21) Application number: 89402369.6

(22) Date of filing: 30.08.89

(51) Int. Cl.5: C 12 N 15/53
C 12 N 15/82, C 12 N 5/10,
A 01 H 5/00, A 01 H 5/10,
C 12 N 9/02

(30) Priority: 02.09.88 GB 8840222

(43) Date of publication of application:
21.03.90 Bulletin 90/12

(84) Designated Contracting States:
AT BE CH DE ES FR GB GR IT LI LU NL SE

(71) Applicant: PLANT GENETIC SYSTEMS, N.V.
Kolonel Bourgstraat 106
B-1040 Brussel (BE)

(72) Inventor: Bowler, Chris
Parklaan 13
B-9000 Gent (BE)

Inze, Dirk
Driesstraat 18
B-9390 Moorsel-Aalst (BE)

Van Camp, Wim
Tulpenlaan 21
B-3540 Zolder (BE)

Alliotte, Thiery
Kortrijksesteenweg 989
B-9000 Gent (BE)

Van Montagu, Marc
120, De Stassartstraat
B-1050 Brussel (BE)

Botterman, Johan
Het Wijngaardeke 5
B-9721 Zevergem-De Pinte (BE)

(74) Representative: Gutmann, Ernest et al
S.C. Ernest Gutmann - Yves Plasseraud 67, boulevard Haussmann
F-75008 Paris (FR)

(54) **Stress-tolerant plants.**

(57) A plant, the nuclear genome of which is transformed with a recombinant DNA sequence encoding a superoxide dismutase which renders the plant stress-resistant. The recombinant DNA sequence also optionally encodes a targeting peptide, fused to the superoxide dismutase, so that the superoxide dismutase is expressed in the cytoplasm of the plant's cells and is subsequently targeted to mitochondria or chloroplasts of the plant's cells or is secreted, via the endoplastic reticulum, from the plant's cells.

EP 0 359 617 A2

Description

# STRESS-TOLERANT PLANTS

This invention relates to DNA molecules and genes coding for metallo-superoxide dismutase enzymes (the "SODs"), particularly plant SODs, particularly a plant manganese superoxide dismutase (the "MnSOD") or a plant iron superoxide dismutase (the "FeSOD"). Production, particularly overproduction, of one or more SOD enzymes can be used to confer on a plant resistance or tolerance to toxic, highly reactive, oxygen species, particularly superoxide anions, produced in the plant's cells under many naturally occurring stress conditions.

This invention also relates to a recombinant gene (the "recombinant SOD gene") which is preferably a chimaeric gene and which contains the following operably linked DNA fragments in the same transcriptional unit: 1) a DNA sequence encoding an SOD (the "SOD gene"), preferably a DNA sequence encoding MnSOD or FeSOD (the "MnSOD gene" or "FeSOD gene", respectively); 2) a promoter suitable for controlling transcription of the SOD gene in a plant cell; and 3) suitable transcription termination and polyadenylation signals for expressing the SOD gene in a plant cell. The recombinant SOD gene optionally contains an additional DNA fragment encoding a targeting peptide (the "targeting sequence") immediately upstream of, and in the same reading frame as, the SOD gene, whereby a plant cell, transformed with the recombinant SOD gene, produces or overproduces a precursor of the SOD having an N-terminal peptide characteristic for: 1) mitochondrial or chloroplast targeting of the SOD within the plant cell; or 2) translocation of the SOD to the lumen of the endoplasmatic reticulum ("ER") of the plant cell for eventual secretion of the SOD out of the plant cell.

This invention further relates to the use of an SOD gene, particularly a recombinant SOD gene, in the production of a transgenic plant having an increased resistance or tolerance to stress conditions which produce highly reactive oxygen species, particularly superoxide anions, in one or more compartments of the plant's cells. This invention relates particularly to the use of the SOD gene for the protection of the plant against naturally occurring stress conditions which are not normally within the control of a farmer (e.g., conditions of soil composition, climate, etc). As a result, the invention provides a means for growing crops in geographical areas in which they could not heretofore be grown with reasonable yields due to such naturally occurring stress conditions.

This invention still further relates to: a cell of a plant (the "transgenic plant cell"), the genome, particularly the nuclear genome, of which is transformed with the recombinant SOD gene; a culture of such cells; a plant (the "transgenic plant") which is regenerated from the transgenic plant cell or is produced from a so-regenerated plant and the genome of which contains the recombinant SOD gene; and the reproductive materials (e.g., seeds) of the transgenic plant. The transgenic plant is resistant or tolerant to stress conditions, particularly naturally occurring stress conditions, which produce highly reactive oxygen species in one or more compartments of the plant's cells, thereby increasing the potential yield and/or quality of crops produced by the plant.

## BACKGROUND OF THE INVENTION

Plants have to be able to cope with a large number of naturally occurring physicochemical stress situations such as drought, waterlogging, high salt concentrations, high or low temperatures, metal excess and metal starvation, as well as biological stress situations such as various pathogens. These stress situations can interfere with normal plant growth and development and consequently, in the case of crop plants, can lower food quality and yield.

It is already known that the production of certain proteins is induced in plants by different stress conditions (Sachs and Ho, 1986). The characteristics of the induced proteins depend upon the actual toxicity effects produced by the stress conditions in the plants. Recently, toxicity due to highly reactive oxygen species has been recognized as an important component of the deleterious effects of a number of stress conditions on plants.

Highly reactive oxygen species are generated in plant cells by the action of various agents such as herbicides (Harbour and Bolton, 1975; Orr and Hogan, 1983), air pollutants (Grimes et al., 1983; Tanaka et al., 1982) such as ozone (MacKay et al., 1987), redox active compounds (Hassan and Fridovich, 1979), heat shock (Lee et al., 1983) and chilling (Clare et al., 1984), and they can cause biologically significant cellular injury. Indeed, superoxide species ($O_2^-$), hydrogen peroxide ($H_2O_2$) and the hydroxyl radical ($OH^-$) can initiate peroxidation of membrane lipids (Mead, 1976), mark proteins for proteolysis (Fucci et al., 1983), cause DNA damage (Brown and Fridovich, 1981; Imlay and Linn, 1988), inhibit photosynthesis (Robinson et al., 1980; Kaiser, 1979) and destroy chlorophyll (Harbour and Bolton, 1978).

Oxygen radicals are also produced under normal conditions in chloroplasts under illumination (Asada et al., 1974), metabolically as products of enzymes (Fridovich, 1978) and in beta-oxidation of fatty acids (Beevers, 1979). All organisms have developed ways to destroy these toxic, highly reactive oxygen species produced under normal conditions. Generally, the superoxide anion is converted to hydrogen peroxide by the action of an SOD in the following reaction:

$$2O_2^- \xrightarrow[2H]{} H_2O_2 + O_2$$

Catalase then catalyzes the decomposition of 2 moles of $H_2O_2$ into 2 moles of $H_2O$ and 1 mole of $O_2$ and thus protects the cells against the noxious $H_2O_2$. In plant cells, peroxidases also provide an important alternative pathway to eliminate $H_2O_2$. SOD and catalase are ubiquitous in aerobic prokaryotic and eukaryotic cells.

SODs are a group of metalloproteins which have been classified according to their metal cofactor (Bannister et al., 1987). Iron enzymes (FeSODs) are present in some prokaryotes and occasionally in plants. Manganese enzymes (MnSODs) are widely distributed among prokaryotic and eukaryotic organisms, and in eukaryotes, they are most often found in the mitochondrial matrix. Copper-zinc enzymes (Cu/ZnSODs) are found almost exclusively in eukaryotes where they are often present in several isoforms. SODs are produced by plant cells as part of their natural defense mechanisms against the toxic effects of highly reactive oxygen species.

The isolation of several cDNA's and genes encoding SODs from various species has been reported (Parker and Blake; 1988; Ho and Crapo, 1988; Bermingham-McDonogh et al., 1988; Marres et al., 1985; Seto et al., 1987; Carlioz et al., 1988). In plants, little is known at the molecular level about SODs. A cDNA clone encoding a cytosolic Cu/ZnSOD has been isolated from maize (Cannon et al., 1987), and a cDNA clone encoding MnSOD from N. plumbaginifolia has been reported (Bauw et al., 1987). However, no sequence data have been published for plant MnSOD or FeSOD genes.

It is known that increased oxygen radicals, produced in plants under oxidative stress conditions, influence the levels of oxygen radical-detoxifying enzymes such as SODs. Resistance against oxygen radical-producing herbicides, such as paraquat (i.e., methyl viologen), has been shown to be correlated with increased levels of enzymes involved in superoxide detoxification in Conyza spec. (Shaaltiel and Gressel, 1987). Similar results have been obtained in Chlorella sorokiniana (Rabinowitch and Fridovich, 1985). Increased Cu/ZnSOD has also been observed in paraquat resistant calluses of Nicotiana tabacum (Furasawa et al., 1984). Paraquat also induces 40% more Cu/ZnSOD in maize whereas only a negligible induction of MnSOD is observed (Matters and Scandalios, 1986). Australian patent AU-A-27461/84 discloses that: paraquat can be used as an efficient weed-killer in postemergence stages, provided that crop plants are made tolerant to its toxic effect; a cDNA clone for human Cu/ZnSOD can be used to identify and isolate a plant DNA segment which carries a plant gene encoding Cu/ZnSOD; and the plant gene encoding Cu/ZnSOD can be inserted, using known expression vectors, into plant cells in order to make paraquat-resistant plants.

The deleterious effects of some air pollutants also seem to be mediated through oxygen radicals. Young poplar leaves, which contain 5 times more SOD than old leaves, are more resistant to $SO_2$ toxicity (Tanaka and Sugahara, 1980). Addition of N-(2-(2-oxo-1-imidazolidenyl)ethyl)-N′-phenylurea ("EDU"), an antiozonant, reduces injury to a plant from ozone. This correlates with an increase in SOD activity in Phaseolus vulgaris (Lee and Bennett, 1982). DDR patent publication 225716 describes the detection of pollution resistant plants by measuring their Cu/ZnSOD content as an indicator of $SO_2$ tolerance.

Green plants are subject to more or less severe damage by combinations of high light intensities with either high or low temperatures. The elevated production of oxygen radicals under these conditions has been implicated as a cause of so-called photooxidative or photodynamic damage. SOD levels in plants are inversely correlated with the extent of the incurred photooxidative damage. This has been reported in ripening tomatoes in which susceptibility to sunscald (a special kind of photodynamic damage) was directly related to SOD activity both under natural and experimental conditions (Rabinowith et al., 1982).

Increased resistance to chilling injury in Chlorella ellipsoidea and to photooxidative death in Plectonema boryanum has also been correlated with SOD activity (Steinitz et al., 1979; Clare et al., 1984).

SOD activity has also been connected to tolerance to hyperoxic or anoxic conditions. The presence of the enzyme in rhizomes of Iris pseudoacorus under anaerobic conditions was seen to be important for its ability to recover from anoxic stress, and the efficacy of recovery was correlated with the SOD level (Monk et al., 1987).

An important naturally occurring stress condition for most plants is the presence of various pathogens which induce so-called "pathogenesis related" ("PR") proteins (Sachs and Ho, 1986; Collinge and Slusarenko, 1987). Major changes in SOD activity apparently take place when plants are attacked by pathogens. Increases in enzymatic activity have been found in susceptible plants while decreases were noticed in resistant ones. Examples are pea roots infected by the cyst nematode Heterodera goettingiana (Arrigoni et al., 1981) and tomato plants infested with Meloidogyne incognita (Zacheo et al., 1982). Plant parasitic fungi of the genera Alternaria and Cercospora are known to produce toxins (altertoxins and cercosporins, respectively) which generate singlet oxygen and superoxide (Daub, 1982, 1987; Daub and Briggs, 1983; Daub and Hangarter, 1983; Hartman et al., 1989). Tobacco plants regenerated from calli selected for high SOD activity were shown to be resistant to cercosporin (Furusawa and Mizuguchi, 1988).

It is also believed possible that SOD is active in the defense of plants against damage from ionizing radiation. At least in the fruit fly Drosophila, a natural genetic polymorphism has been found in regard to SOD. Flies with the greatest resistance against ionizing radiation carry an allele which codes for an SOD enzyme with a higher specific activity (Peng et al., 1986).

Reduced oxygen species also appear to play a key role in the aging process of plants (Munkres et al., 1984).

SUMMARY OF THE INVENTION

In accordance with this invention, a plant MnSOD gene and FeSOD gene and the MnSOD and FeSOD, which can be produced by expression of these genes in procaryotic or eucaryotic cells, particularly plant cells, are provided.

Also in accordance with this invention, a recombinant SOD gene, preferably a chimaeric recombinant SOD gene, is provided for stably transforming a plant cell genome, containing the following operably linked DNA fragments: 1) a SOD gene; 2) a promotor capable of directing expression of the SOD gene in a plant cell; and 3) suitable 3′ transcription regulation signals. Preferably, the recombinant SOD gene also contains a targeting sequence which is located between the promoter and the SOD gene, which is in the same reading frame as, and fused to, the SOD gene, and which codes for a mitochondrial or chloroplastic targeting peptide of the plant cell or for a targeting peptide for translocation to the lumen of the ER of the plant cell, so that a fusion protein containing the targeting peptide and the SOD can be synthesized in the cytoplasm of the plant cell and so that the fusion protein will be processed by the plant cell whereby the SOD is translocated into mitochondria or chloroplasts of the plant cell or into the lumen of the ER for secretion out of the plant cell.

Further in accordance with this invention are provided transgenic plant cells, cultures of the plant cells, and transgenic stress-resistant plants regenerated from the cells, which have stably incorporated into their genome, preferably their nuclear genome, one or more of the recombinant SOD genes, so that at least one SOD is secreted from the cells or is expressed or overexpressed in the cytosol, the mitochondria and/or the chloroplasts of the cells.

DESCRIPTION OF THE INVENTION

The plant MnSOD gene of the present invention can be identified, isolated and characterized using methods well known to those skilled in the art. The aminoterminal sequence of proteins electroblotted on support material after two dimensional gel electrophoresis can be determined in a straightforward manner by gas-phase sequencing of the immobilized proteins (Bauw et al., 1987). In this way the amino-terminal aminoacid sequence of the MnSOD of Nicotiana plumbaginifolia was determined. The construction of an oligonucleotide probe specific for this aminoacid sequence then allows the isolation of the cDNA encoding the MnSOD from a cDNA library of Nicotiana plumbaginifolia. The cDNA can subsequently be manipulated and sequenced using standard methods (Maniatis et al., 1982; Maxam and Gilbert, 1980). The complete aminoacid sequence of the plant MnSOD can then be deduced from the cDNA sequence. It goes without saying that, not only the specific cDNA sequences of Figure 1, 2 and 3, but also all DNA sequences coding for proteins with the deduced amino acid sequences of Figure 1, 2, and 3 and their equivalents with MnSOD activity, fall within the scope of this invention.

The isolated cDNA of the MnSOD gene contains not only the complete reading frame of 204 aminoacids comprising the mature active MnSOD but also a transit peptide-encoding sequence of 24 aminoacids which targets the enzyme into the mitochondria as deduced from the general properties of mitochondrial transit peptides (Schatz, 1987). The complete nucleotide and amino acid sequence of the cDNA coding for the mitochondrial transit peptide and MnSOD is shown in Fig. 1.

The available data on plant FeSOD genes suggest that they are fairly different from procaryotic FeSOD genes. However, plant FeSOD genes can be identified by complementation of a SOD deficient E. coli strain. In this procedure comprised of individual steps well known to those skilled in the art, a cDNA library of Nicotiana plumbaginifolia can be cloned in an E.coli expression vector, such as pUC18 (Yannisch-Perron et al., 1985), and the resulting plasmids can be used to transform a SOD deficient E. coli strain such as that described by Carlioz and Touati (1987) by electroporation in order to obtain high transformation efficiency. Colonies that are able to grow aerobically on minimal medium and that are synthesizing SOD can be identified by staining for SOD activity of total cellular protein separated by polyacrylamide gel electrophoresis. The cDNA inserts in the expression vector can then be further characterized by restriction analysis and/or hybridization studies. Inserts that are thought to be coding for a FeSOD (i.e., that do not hybridize with known plant Cu/ZnSODs and MnSOD and that direct the production of an active SOD that is resistant to KCN and sensitive to $H_2O_2$) are selected for further characterization by means of DNA sequencing (Sanger et al., 1977). The aminoacid sequence of the FeSOD can then be inferred from the obtained nucleotide sequence. Using this procedure a cDNA coding for a FeSOD from Nicotiana plumbaginifolia, characterized by the sequence in Fig. 5, could be obtained.

Although the plant SOD genes, particularly the plant MnSOD and FeSOD genes, are the preferred SOD genes in the recombinant SOD genes of this invention, other genes coding for enzymes with SOD activity, such as procaryotic SOD genes and cDNAs derived from eucaryotic SOD genes, also can be used. In this regard, the selection of the SOD gene is not believed to be critical, and a particular plant cell can be transformed with a recombinant SOD gene containing either: a foreign SOD gene which will produce its encoded SOD in the cell; or an endogenous SOD gene which will provide overproduction of its encoded SOD in the cell. For example, suitable foreign SOD genes can encode: the procaryotic and eucaryotic Cu/ZnSODs listed by Getzoff et al. (1989); the procaryotic and eucaryotic MnSODs listed in Bowler et al. (1989a); and the procaryotic FeSODs described by Carlioz et al., 1988) and Parker and Blake (1988).

For constructing a recombinant SOD gene which can be expressed in a transformed plant cell, suitable promoters are known which can be provided upstream (i.e., 5′) of a SOD gene, and the selection of a promoter is not believed to be critical. In this regard, a particular plant cell can be transformed with a recombinant SOD

gene containing either a foreign or an endogenous promoter suitable for directing expression or overexpression of the SOD gene. Suitable foreign constitutive promoters include: the promoter of the cauliflower mosaic virus ("CaMV") isolates CM1841 (Gardner et al., 1981) and CabbB-S (Franck et al., 1980) [the "35S promoter"] which directs constitutive expression of heterologous genes (Odell et al., 1983); a related promoter (the "35S3 promoter") which can be isolated from the CaMV isolate CabbB-JI (Hull and Howell, 1978) and which differs from the 35S promoter in sequence and in its greater activity in transgenic plants (Harpster et al., 1988); and the TR1′ and the TR2′ promoters which drive the expression of the 1′ and 2′ genes, respectively, of the T-DNA (Velten et al., 1984) and are wound-induced promoters. Suitable organ-specific, tissue-specific and/or inducible foreign promotors are also known (Kuhlemeier et al., 1987 and the references cited therein) such as the promoter of the 1A small subunit gene of 1,5 ribulose bisphosphate carboxylase (Rubisco) of Arabidopsis thaliana (the "ssu promoter") which is a light-inducible promoter (European patent 242,246) active only in photosynthetic tissue. Other organ-specific, tissue-specific and inducible promoters can be isolated from cell- or tissue-or organ-specific genes and from genes specific for particular developmental stages (Goldberg, 1988) by the screening of plant genomic librairies with specific cDNAs, using techniques as disclosed, for example, in European patent applications 89402224.3 and 89401194.9.

For constructing a recombinant SOD gene which can be expressed in a transformed plant cell, suitable transcription termination and polyadenylation signals are known which can be provided downstream (i.e., 3′) of an SOD gene, and the selection of such 3′ transcription regulation signals is not critical. In this regard, a particular plant cell can be transformed with a recombinant SOD gene containing either foreign or endogeneous transcription termination and polyadenylation signals suitable for obtaining expression or overexpression of the SOD gene. For example, the foreign 3′ untranslated ends of genes, such as gene 7 (Velten and Schell, 1985), the octopine synthase gene (Gielen et al., 1983) and the nopaline synthase gene of the T-DNA region of Agrobacterium tumefaciens Ti-plasmid (European patent application 89402224.3), can be used.

By "recombinant" with regard to the recombinant SOD gene of this invention is meant that its operably linked SOD gene, promoter and 3′ transcription regulation signals, together with any targeting sequence, can be introduced in a plant genome by artificial means (e.g., by Agrobacterium-mediated gene transfer) and are then (in the plant genome) not in their natural genomic environment (i.e., are not surrounded in the plant genome by their naturally surrounding DNA sequences).

By "chimaeric" with regard to the chimaeric recombinant SOD gene of this invention is meant that its SOD gene: 1) is not naturally found under the control of its promoter and/or 2) is not naturally found fused to, and in the same reading frame as, its targeting sequence. Examples of chimaeric recombinant SOD genes of this invention comprise: an SOD gene of bacterial origin under the control of a promoter of plant origin; and a SOD gene of plant origin under the control of a promoter of viral origin and fused to a signal sequence encoding a transit peptide of plant origin.

For constructing a recombinant SOD gene which can be expressed in a transformed plant cell, preferably in its cytoplasm, by the production or overproduction of an SOD, followed by translocation to the cell's mitochondria, chloroplasts and/or lumen of the cell's endoplasmatic reticulum ("ER") for eventual secretion from the cell, suitable targeting sequences encoding targeting peptides are known. Selection of such targeting sequences is not believed to be critical, and a particular plant cell can be transformed with a recombinant SOD gene containing either a foreign or an endogeneous targeting sequence which will provide translocation or secretion of the expression product of the SOD gene.

By "targeting peptide" is meant a polypeptide fragment which is normally associated in a eucaryotic cell with a chloroplast or mitochondrial protein or subunit of the protein or with a protein translocated to the ER and which is produced in a cell as part of a precursor protein encoded by the nuclear DNA of the cell. The targeting peptide is responsible for the translocation process of the nuclear-encoded chloroplast or mitochondrial protein or subunit into the chloroplast or the mitochondria or the lumen of the ER. During the translocation process, the targeting peptide is separated or proteolytically removed from the protein or subunit. A targeting sequence can be provided in the recombinant SOD gene of this invention for providing a targeting peptide to translocate an expressed SOD within a transformed plant cell as generally described in European patent applications 85402596.2 and 88402222.9. A suitable targeting peptide for transport into chloroplasts is the transit peptide of the small subunit of the enzyme RUBP carboxylase (European patent application 85402596.2), but other chloroplast transit peptides, such as those listed by Watson (1984), can also be used. A suitable mitochondrial targeting peptide is the transit peptide naturally associated with a plant MnSOD as shown in Figure 1, but other mitochondrial transit peptides, such as those described by Schatz (1987) and listed by Watson (1984), can be used. Suitable targeting peptides that can translocate an SOD to the lumen of the ER of a plant cell are, for instance, the signal peptides described by Von Heijne (1988) and listed by Watson (1984).

In accordance with this invention, any SOD gene, preferably a plant SOD gene, particularly a plant MnSOD or FeSOD gene, can be used to produce a transformed plant with increased tolerance or resistance to the toxic effects of highly reactive oxygen species, particularly superoxide anions, produced in one or more compartments of its cells as a result of certain stress conditions, oxidation during or after harvest, or senescence. Preferably, the resulting SOD is produced in, or transported to, mitochondria, chloroplasts, cytosols or other specific sites in or out the plant cells, appropriate to combatting the effects of the toxic highly

reactive oxygen species. In this regard, the targeting sequence encoding a targeting peptide in the recombinant SOD gene permits the SOD expression product to be targeted to one or more cell compartments in or out of the plant cells where stress-produced, highly reactive, oxygen species pose a particular problem as discussed below.

The recombinant SOD gene of this invention, which is preferably chimaeric, can be used to produce transgenic plant cells and transgenic plants in which the SOD gene is expressed or preferably overexpressed. This confers on these cells and plants an increased resistance to the toxicity of highly reactive oxygen species, particularly superoxide radicals. Since the formation of highly reactive oxygen species is likely to occur at different sites within the plant cells, depending upon the nature of the stress to which the cells are subjected, it is important to be able to direct expression of the SOD gene into one or more compartments of the cells containing such sites. Directing SOD gene overexpression into chloroplasts can be important for protection against stress induced by conditions such as high light intensities in combination with high or low temperatures (Bowles, 1984) or the presence of paraquat (Shaaltiel and Gressel, 1987), which generate highly reactive oxygen species in these organelles. The targeting of SOD overexpression into mitochondria aims at protecting plants against deleterious effects of highly reactive oxygen species generated in these organelles, for example by plant pathogens which can cause increased respirational activity in mitochondria. SOD overexpression in the cytosol can protect plants against the effects of highly reactive oxygen species generated in this cell compartment, thereby leading, for example, to increased storage life of the plants and to their fruit having increased tolerance to bruising. SOD secretion from plant cells can provide increased resistance to reactive oxygen species outside the plant cells, such as are caused by infections of pathogens responsible for the production of highly reactive oxygen species (e.g., by the fungus Cercospora) and by air pollutants (e.g., ozone and $SO_2$). It is also believed that SOD-overexpressing plants may have a prolonged lifespan due to their higher level of protection against toxic oxygen species.

A transgenic plant of this invention can be produced by the introduction of the recombinant SOD gene into a cell of the plant, followed by regeneration of the plant, using known techniques. A disarmed Agrobacterium tumefaciens Ti-plasmid can be used for transforming the plant cell with the recombinant SOD gene using procedures described, for example, in European patent publications 116718 and 270822, PCT publication WO 84/02913 and European patent application 87400544.0. Preferably, the Ti-plasmid contains the recombinant SOD gene between its border sequences or at least located to the left of the right border sequence of the T-DNA of the Ti-plasmid. Such a Ti-plasmid can be used for the transformation of all plants susceptible to Agrobacterium infection, such as Nicotiana plumbaginifolia, Arabidopsis thaliana Nicotiana tabacum, Solanum tuberosum, Lycopersicum esculentum, Medicago sativa and Beta vulgaris. Other techniques can be used to transform these and other plants, such as: direct gene transfer (as described, for example, in European patent publication 223247), pollen mediated transformation (as described, for example, in European patent publication 270356, PCT publication WO 85/01856 and European patent publication 275069), in vitro protoplast transformation (as described, for example, in US patent 4684611), plant RNA virus-mediated transformation (as described, for example, in European patent publication 67553 and US patent 4407956) and liposome-mediated transformation (as described, for example, in US patent 4536475).

The resulting transgenic plant can be used in a conventional plant breeding scheme to produce more transgenic plants with the same characteristics or to introduce the recombinant SOD gene in other varieties of the same or related plant species. Reproductive materials (e.g., seeds), which are obtained from the transgenic plants, contain the recombinant SOD gene as a stable genomic insert.

The production of transgenic plants, having two or more recombinant SOD genes which are stably integrated in their genomes, particularly their nuclear genomes, and which target the expression or overexpression of one or more SODs into several different compartments of the plants' cells at once (e.g., with targeting sequences encoding different targeting peptides), can provide additional benefits in stress tolerance or resistance. Such a stress-resistant plant can be obtained in several ways, such as by: several independent transformations of the same plant with different recombinant SOD genes; a single transformation with a vector containing two or more recombinant SOD genes in tandem; or crossing two plants, each of which has already been transformed with different recombinant SOD genes. In order to be able to adequately select plants with two recombinant SOD genes, it is advisable that each recombinant SOD gene contain, within the same genetic locus, a different selectable marker gene. Selectable marker genes that can be used for this purpose are, for instance, the neo gene coding for neomycin phosphotransferase (Reiss et al., 1984) and the bar gene coding for phosphinotricin acetyltransferase (as described in European patent publication 242246).

The transgenic plant cells, cell cultures and plants of this invention can also be used to produce an overexpressed SOD, especially a plant SOD, particularly a plant MnSOD or FeSOD. In a conventional manner, the so-produced SOD can be recovered from the plant cells and used as an anti-oxidative food additive, an anti-inflammatory agent in mammals, or a therapeutic agent in mammals for certain pathological conditions that generate superoxide radicals or for prevention of ischemic injuries (See Bannister et al., 1987).

An SOD of this invention can also be produced, preferably as a secreted protein, by linking an SOD gene to suitable expression signals such as appropriate promoter sequences, secretion targeting sequences, ribosome binding sites, start and stop codons and transcription regulation sequences for prokaryotic and/or eukaryotic cells. The so-linked SOD gene can then be introduced into a host, such as a prokaryotic or eukaryotic cell, in which the SOD gene can be expressed and/or replicated. The host can then be cultured, after which any SOD, produced by it, can be recovered. Such a process for expressing and/or replicating an

SOD gene can be carried out in a conventional manner, using known expression vectors and host environments (See Winnacker, 1987 and the references cited therein), as well as known replicons which can carry the SOD gene so that the gene is expressed, as well as propagated, in a host.

The Examples, which follow, illustrate the invention. In the Examples, reference is made to the accompanying drawings in which:

Figure 1: The aminoacid sequence (bottom) of the mature MnSOD of Nicotiana plumbaginifolia, linked to the mitochondrial transit peptide, and the corresponding DNA sequence coding for this amino acid sequence (top). This DNA sequence corresponds to the cDNA as comprised in plasmid pSOD-1. The numbering refers to the nucleotides of the open reading frame comprising the mitochondrial transit peptide and the mature MnSOD. The N-terminus of the MnSOD is indicated by an arrow.

Figure 2: The amino acid sequence (bottom) of the mature MnSOD of Nicotiana plumbaginifolia and the corresponding DNA sequence coding for this amino acid sequence (top) used in Example 4. The real N-terminus of the MnSOD is indicated by an arrow; the preceding amino acids are derived from the cloning procedure.

Figure 3: The amino acid sequence (bottom) of a polypeptide with MnSOD activity of Nicotiana plumbaginifolia, linked to the chloroplast transit peptide, and the corresponding DNA sequence coding for this amino acid sequence (top) used in Example 5. The N-terminus of the mature MnSOD is indicated by an arrow. The expected cleavage site of the transit peptide is indicated by a double arrow.

Figure 4a: Restriction map of plasmid pDE9 containing the 35S3 promotor from CaMV isolate CabbB-JI.

Figure 4b: DNA sequence of the 35S3 promoter fragment of CaMV isolate CabbB-JI.

Figure 5: The amino acid sequence (bottom) of the mature FeSOD of Nicotiana plumbaginifolia, as cloned in the PstI site of plasmid PUC18, and the corresponding DNA sequence coding for this amino acid sequence (top) used in Example 12. The sequence is given for the lacZ initiation codon of pUC18. The start of the FeSOD sequence is indicated by an arrow.

Figure 6: Construction of pEX1SOD of Example 3.

Figure 7: Construction of pEX3SOD of Example 4.

Figure 8: Construction of pEX4SOD of Example 5.

Figure 9: Construction of pEX5SOD of Example 6.

Figure 10: Expression of MnSOD in transgenic plant calli.

Figure 11: Percent weight change in relation to paraquat concentration for leaf discs derived from transgenic (T16-213 (11A) and T16-202 (11B) overexpressing MnSOD in the chloroplasts) and control (T17-50) N. tabacum PBD6 (Crosses: transgenic plants; open circles: control plants).

Figure 12: Percent bleaching of chlorophyll pigments (measured at 664nm) in relation to paraquat concentration for leaf discs derived from transgenic (T16-213 (12A) and T16-202 (12B) overexpressing MnSOD in the chloroplasts) and control (T17-50) N. tabacum PBD6 (Crosses: transgenic plants; open circles: control plants).

Figure 13: Percent bleaching of chlorophyll in relation to initial chlorphyll content (measured as $E_{ini}$ per 35 mg of leaf tissue) for leaf discs derived from various transgenic and control N. tabacum PBD6 (treated with 50uM paraquat for 24 hrs.)

Figure 14: TBA reactivity (TBAR) in relation to paraquat concentration for various transgenic and control N. tabacum PBD6. Expression levels of MnSOD for each plant are indicated.

The abbreviations used in the Figures and in the Examples are:

Gluc: Glucose
Sucr: Sucrose
Mann: Mannitol
mit-tp: Mitochondrial transit peptide encoding sequence
cp-tp: Chloroplast transit peptide encoding sequence
ori: origin of replication (Ori-1: of pBR322 plasmid of E. coli, Ori-2: of pVS1 plasmid of Pseudomonas aeruginosa).
Sm: Streptomycin resistance gene
Cont: Control
MV: Methyl Viologen (Paraquat)
Ch+, Ch++, Ch+++: Expression levels of MnSOD in chloroplasts of transgenic plants
M+, M++, M+++: Expression levels of MnSOD in mitochondria of transgenic plants
C+: Expression levels of MnSOD in cytosol of transgenic plants
Sp: Spectinomycine resistance gene
RB-LB: Right and left borders of T-DNA of Agrobacterium tumefaciens
35S: 35S promotor of CaMV isolate CM1841.
35S3: 35S promotor of CaMV isolate CabbB-JI
3'g7: polyadenylation signal sequence of T-DNA gene 7
pnos: promoter of the nopaline synthase gene of A. tumefaciens.
3' ocs: polyadenylation signal sequence of the octopine synthase gene of A. tumefaciens.
neo: neomycine phosphotransferase II gene
MnSOD: manganese superoxide dismutase gene of Nicotiana plumbaginifolia.
cip: Calf intestinal alkaline phosphatase

pSSU: promoter of the 1A small subunit of Rubisco of Arabidopsis thaliana.

Unless otherwise specified in the Examples, all procedures for making and manipulating recombinant DNA are carried out by the standardized procedures described by Maniatis et al., 1982. The following plasmids and vectors, used or prepared in the Examples, have been deposited in the Deutsche Sammlung Für Mikroorganismen und Zellkulturen ("DSM"), Mascheroder Weg 1B, Braunschweig, Federal Republic of Germany under the provisions of the Budapest Treaty:

| Plasmid or Vector | DSM Accession No. | Date of Deposit |
|---|---|---|
| pSC1701A2 | DSM 4286 | 22 Oct. 1987 |
| pGSC1700 | DSM 4469 | 21 Mar. 1988 |
| pEX1SOD | DSM 4695 | 8 Jul. 1988 |
| pEX3SOD | DSM 4696 | 8 Jul. 1988 |
| pEX4SOD | DSM 4692 | 8 Jul. 1988 |
| pEX5SOD | DSM 4693 | 8 Jul. 1988 |

Example 1: Isolation of superoxide dismutase cDNA clone from Nicotiana plumbaginifolia.

Cell suspension cultures were initiated from homozygous N. plumbaginifolia plants. Total protein extracts were separated by two-dimensional polyacrylamide gel electrophoresis, and the separated proteins were recovered by electroblotting onto membranes that allow direct gas-phase sequencing analysis of the immobilized proteins (Bauw et al., 1987). Proteins were visualized by U.V. illumination after treatment with fluorescamine. Protein spots were removed by scissors and stored at -20°C (Bauw et al., 1987). One of the isolated proteins had the following $NH_2$- terminal sequence:

LQTFSLPDLPYDXGALEPAI?GD

in which "?" is an unknown residue and "X" indicates a modified residue (for standard abbreviations of amino acids see Singleton and Sainsbury, 1987). Comparison of the protein sequence with published eukaryotic protein sequences within the National Biomedical Research Foundation Protein Sequence Data Bank (Release 9) [U.S.A.] showed partial homology with the human Mn superoxide dismutase (Harris and Steinman, 1977) and with the corresponding enzyme of Saccharomyces cerevisiae (Harris and Steinman, 1977). The determined N-terminal sequence was, therefore, presumed to belong to a plant MnSOD. To obtain the cDNA clone encoding the complete enzyme, an oligonucleotide was designed to match part of the N-terminal amino acid sequence. This was synthesized with a deoxyinosine at ambiguous codon positions (Ohtsuka et al., 1985; Takahashi et al., 1985) and used as probe to screen a cDNA library from a N. plumbaginifolia cell suspension culture depleted 14 days for cytokinin (Bauw et al., 1987). The procedure to construct the cDNA library was as described in Gubler and Hoffman (1983). The oligonucleotide sequence was:

5′-CCITAIGAITAIGGIGCICTIGAICCIGC-3′

$5X10^4$ clones from the cDNA library were hybridized with the oligonucleotide at 40°C. Twelve clones showed a positive signal. One clone "pSOD1" was selected for further analysis. The pSOD1 cDNA was sequenced on both strands according to the method of Maxam and Gilbert (1980). The entire sequence of the 996 bp cDNA insert is shown Fig. 1, with its flanking G/C homopolymer tails added during the cloning procedure. It contains one continuous open-reading frame, corresponding to 228 amino acids. The sequence homologous to the oligonucleotide probe is underlined. The cDNA clone also contains a mitochondrial leader sequence of 24 amino acids upstream from the mature protein (Fig. 1). The amino acid sequence, deduced from the cDNA sequence, is written in Fig. 1 below the nucleotide sequence in the one-letter code. The amino acid sequence starting from amino acid 25 (indicated by arrow) is completely homologous to the previously determined N-terminal amino acid sequence of the mature protein. The molecular weight calculated from the cloned sequence is 22.8 kD for the mature MnSOD and 25.5 kD for the transit peptide-MnSOD preprotein.

A comparison between the N. plumbaginifolia mature protein and the MnSOD of bacteria, yeast and humans shows considerable homology (Bowler et al., 1989a).

The comparative homologies show that the plant MnSOD is more closely related to human and yeast, than to bacterial, MnSOD. However, a posteriori comparative analysis of the respective genes shows that isolation of the plant MnSOD would have been difficult using a cloned bacterial, yeast or human SOD gene fragment as a probe for screening a plant cDNA library.

The presence in the signal sequence of the five arginine residues distributed among uncharged amino acids, the absence of acidic residues, and the occurrence of hydroxylated amino acids such as serine and threonine are typical for a leader sequence for translocation to the mitochondrial matrix (Schatz, 1987). A mitochondrial location is consistent with data from analysis of subcellular fractions.

Example 2: Expression of MnSOD in N. plumbaginifolia plants.

MnSOD was identified as a highly abundant protein in cell suspension cultures of N. plumbaginifolia. Northern analysis on total RNA, isolated from different tissues of N. plumbaginifolia plants, with the pSOD1 cDNA as probe revealed great variations in steady state mRNA. Plants were sterile-grown at 25°C on

Murashige and Skoog ("MS") medium (Murashige and Skoog, 1962). They were grown with a 16-hour light/ 8-hour dark cycle on solid MS medium containing 0.1 M sucrose. Cell suspension cultures were grown in the dark, in liquid medium with 0.1 M sucrose and supplemented with 0.5 mg/l naphthalene acetic acid ("NAA") and 0.1 mg/l 6-benzylaminopurine ("BAP"). They were subcultured every 3 days. Total RNA was prepared according to Jones et al. (1985). 12 ug RNA was denatured in formaldehyde, electrophoresed, transferred to nylon membranes and hybridized with $^{32}$p-labeled RNA-probes corresponding to the HpaI-Hind III fragment of pSOD1. Expression was very weak in leaves of intact plants, 2- to 3-fold higher in roots, and 50 times higher in dark-grown cell suspension cultures (Bowler et al., 1989a). These differences in expression were not due to a switch-off of photosynthesis, since exposure of whole plants to the dark did not result in increased expression of MnSOD. Treatment with several concentrations of paraquat resulted in very minor increases of MnSOD mRNA in leaves (Bowler et al., 1989a). Incubation of leaf discs for 48 hours in the dark in liquid medium yielded induced MnSOD mRNA levels comparable to those in cell suspension cultures. Incubation of leaf discs in pure water caused only a 2-3 fold induction, which is similar to the effect of wounding whole plants by cutting their leaves in several places (Bowler et al., 1989a).

Sucrose was found to be a crucial factor for induction of MnSOD RNA. The effect was greatest in the presence of salts and showed a linear dose-response at sucrose concentrations ranging from 0.001 till 0.2 M. The highest level of expression was reached after 48 hrs incubation. Combinations of salts delivering iron, manganese, copper and zinc ions in the presence of sucrose showed that the induction was not due to these salts in particular. The increase in MnSOD mRNA was also possible by induction with glucose, but not with mannitol (Bowler et al., 1989a).

Levels of MnSOD protein were measured by assaying SOD activity. Leaf discs were incubated for various time periods in MS + 0.1 M sucrose supplemented with 0.5 mg/l NAA and 0.1 mg/l BAP and subsequently homogenized in an equal volume of cold extraction buffer (50 mM potassium phosphate, pH 7.8, 0.1 % ascorbate, 0.05 %, beta-mercaptoethanol, 0.2 % Triton X-100) and centrifuged at 13,000 rpm for 12 minutes. Protein samples were separated on non-denaturing 10 % polyacrylamide gels run at 120 V constant voltage. SOD activity was localized on these gels using the in situ staining technique according to Beauchamp and Fridovich (1971). Inhibition studies with $H_2O_2$ and KCN (Bridges and Salin, 1981) revealed the upper band to be MnSOD and the lower band (only present in samples containing more than 100ug protein) a Cu/ZnSOD. Semi-quantitative data were obtained by loading several concentrations (10-200 ug) of total protein on the gel. Change of MnSOD activity was best visualized on samples containing 50 ug protein. In relation to the mRNA profile, a similar, albeit delayed, induction pattern was obtained at the protein level (Bowler et al., 1989a). The increase in SOD activity caused by addition of exogenous sugars appeared to be entirely due to expression of MnSOD, since the Cu/ZnSOD in the extracts showed no significant alteration in expression level (Bowler et al., 1989a).

Example 3: Construction of pEX1SOD comprising the MnSOD cDNA under the control of the CaMV 35S promoter (Fig. 6)

The MnSOD cDNA was isolated from pSOD1 of Example 1 as a 910 bp HpaI-SmaI restriction fragment. This fragment was cloned in the vector PGSJ780A derived from pGSC1701A2. Plasmid pGSJ780A contains the 35S promoter fragment (Odell et al., 1983) from CaMV isolate CM1841 (Gardner et al., 1981) and the 3′ untranslated region of T-DNA gene 7 (Velten and Schell, 1985) as well as a chimaeric cassette for plant transformation selection. This vector was digested with ClaI and treated with Klenow DNA polymerase to generate blunt ends. This yielded the plasmid "pEX1SOD" which contains, between T-DNA border sequences, two chimaeric genes:

The first chimaeric gene contains:

- the CaMV 35S promoter,
- the MnSOD gene consisting of the coding sequences for both the mitochondrial transit peptide and the mature MnSOD (Figure 1), and
- the 3′ end of T-DNA gene 7 used for correct polyadenylation of the mRNA;

The second chimaeric gene contains:

- the nopaline synthase promoter,
- the neo gene encoding neomycin phosphotransferase II, and
- the 3′ end of octopine synthase,

and serves as a selectable marker during plant transformation (Hain et al., 1985). Since the plasmid contains a plant MnSOD gene with its own transit peptide sequence under the control of the constitutively expressed 35S promotor, targeting to the mitochondria is expected.

Example 4: Construction of pEX3SOD comprising the MnSOD cDNA sequence lacking the mitochondrial signal peptide sequence under control of the CaMV 35S promoter (Fig. 7)

The SacII site, which is present at the transit peptide cleavage site of the MnSOD clone in pSOD1 from Example 1, was converted to a BglII site as follows. pSOD1 was digested with SacII, blunt ended with Klenow DNA polymerase and ligated with octamer BglII linkers. This yielded the plasmid "pSOD-B". The BglII-BamHI fragment from pSOD-B, containing the SOD cDNA clone, was isolated and cloned in the BamHI site of pGSJ780A. This yielded the plasmid "pEX3SOD" which contains, between T-DNA border sequences, two chimaeric genes:

The first chimaeric gene contains:
- the CaMV 35S promoter,
- the MnSOD encoding sequence without transit peptide encoding sequence (Figure 2), and
- the 3′ end of gene 7.

The second chimaeric gene contains:
- the nopaline synthase promoter,
- the neo gene, and
- the 3′ end of octopine synthase,

and serves as a selectable marker for plant transformation. The MnSOD cDNA, under the control of the 35S promoter, will yield a cytoplasm localized MnSOD, constitutively expressed in all tissues.

Example 5: Construction of pEX4SOD comprising the MnSOD cDNA sequence and an upstream chloroplast transit peptide sequence under control of the CaMV 35S promoter (Fig. 8)

The BglII-BamHI fragment was isolated from pSOD-B of Example 4 and cloned in the BamHI site of pKAH1 yielding pKAH1-SOD. pKAH1 contains the transit peptide sequence of the Rubisco pea small subunit ("ssu") gene SS3.6 (Cashmore, 1983). pKAH1-SOD contains the SOD cDNA clone (without mitochondrial transit peptide) fused to the chloroplast transit peptide ("tp") sequence of the ssu gene under the control of the 35S promoter of CaMV isolate CM1841. Finally, the BglII-BamHI fragment from pKAH1-SOD, carrying the 35S-tp-SOD cassette, was cloned in the BamHI site of pGSC1702 yielding the plasmid "pEX4SOD". pGSC1702 is derived from pGSC1700 and contains the 3′ untranslated region of T-DNA gene 7 as well as a chimaeric cassette for transformation selection. The chimaeric construct in pEX4SOD differs from that in pEX3SOD in having a chloroplast transit peptide sequence at the N-terminus of the MnSOD cDNA coding sequence as shown in Figure 3. In this regard, the mitochondrial transit peptide sequence has been replaced by a chloroplast transit peptide sequence in order to constitutively express a MnSOD which is targeted to chloroplasts.

Example 6: Construction of pEX5SOD comorisino the MnSOD cDNA under the control of the Arabidopsis Rubisco small subunit promoter (Fig. 9)

The BglII-EcoRI fragment of pSOD-B, containing the SOD fragment, was cloned into the plasmid pC23, digested with BglII and EcoRI, yielding the plasmid "pC23SOD-B". pC23 was obtained from pC22 (Simoens et al., 1986) by the downstream extension of the polylinker ApaI-SmaI-EcoRI-XbaI by a StuI, a HindIII and a BglII site. As described in European patent 242,246, a Rubisco small subunit gene ("ssu") promoter from Arabidopsis thaliana is contained in plasmid pATS3 as a 1.5 kb EcoRI-SphI fragment. The ssu promoter fragment was cloned into a vector (designated as pGS1400) so that it could be excised as a BglII-BamHI fragment. This BglII-BamHI fragment was cloned in the BglII site of pC23SOD-B, yielding the plasmid "pC23SSUSOD". Finally, the BglII-BamHI fragment from pC23SSUSOD, containing the ssu promoter fragment fused to the SOD cDNA clone, was isolated as a BglII-BamHI fragment and inserted into the BamHI site from pGSC1702 upstream of the 3′ end of gene 7 yielding the plasmid "pEX5SOD". This plasmid contains, between T-DNA border sequences, two chimaeric constructs:

One chimaeric gene contains:
- the Rubisco small subunit 1A promoter from Arabidopsis thaliana,
- the MnSOD mature protein coding sequence, and
- the 3′ end of gene 7.

The second chimaeric gene contains:
- the nopaline synthase promoter,
- the neo gene, and
- the 3′ end of octopine synthase,

and serves as a selectable marker during plant transformation. Since the leaderless MnSOD cDNA sequence is under the control of a ssu gene promoter, light regulated expression of the MnSOD in the cytoplasm is expected.

Example 7: Chimaeric constructs under the control of the 35S3 promoter from the CaMV isolate CabbB-JI

The 35S3 promoter of CaMV isolate CabbB-JI (Hull and Howell, 1978) was cloned in pUC19 (Yannish-Perron et al., 1985) yielding pDE9 (Fig. 4A) and sequenced. The sequence of the 35S3 promoter fragment as contained in PDE9 is presented in Fig. 4B. The NcoI site of the promoter fragment was created at the first ATG codon occurring in the 35S3 RNA transcript by site directed mutagenesis using the pMa5-8 and pMc5-8 plasmids (European patent application 87402348.4) and the gapped duplex procedure of Stanssens et al. (1987).

Analysis of the nucleotide sequence of the 35S3 promoter showed that it differs from that of the 35S promoter from CaMV isolate CabbB-S (Franck et al., 1980) and from CaMV isolate CM1841 (Gardner et al., 1981). Moreover, some chimaeric constructs with the 35S3 promoter have shown greater activity in transgenic plants than with the 35S promoter from CabbB-S (Harpster et al., 1988). The 35S3 promoter can be clearly distinguished from the other two 35S promoters, mentioned above, by the absence of an EcoRV site due to a single nucleotide substitution, immediately ahead of the TATA box.

Using standard recombinant DNA techniques, the 35S promoter in the plant expression vectors pEX1SOD,

pEX3SOD and pEX4SOD of Examples 3, 4 and 5 is replaced by the 35S3 promoter to yield three additional plant expression vectors "pEX6SOD", "pEX7SOD" and "pEX8SOD", respectively.

Example 8: Plant transformation and regeneration

Using well-known techniques as described in European patent publication 116718 and European patent application 87400544.0, plants cells are transformed, as described below, with the plant expression vectors pEX1SOD, pEX3SOD, pEX4SOD, pEX5SOD, pEX6SOD, pEX7SOD and pEX8SOD from Examples 3-5 and 7.

The plant expression vectors are mobilized into the Agrobacterium tumefaciens recipient strain C58C1Rif (pGV2260) according to the procedure described by Deblaere et al. (1985). These strains are then used for the transformation and regeneration of: Arabidopsis thaliana according to the procedure of Valvekens et al., (1988); and Nicotiana plumbaginifolia and Nicotiana tabacum PBD6 according to the leaf disc transformation procedure of De Block et al. (1987). Transformed shoots of N. plumbaginifolia and Nicotiana tabacum are regenerated into whole plants according to the methods of Ellis et al. (1988) and De Block et al. (1987), respectively.

Example 9: Analysis of transgenic plants

The plants of Example 8, transformed with pEX1SOD, pEX3SOD and pEX4SOD, were analyzed for the expression of recombinant SOD genes as follows.

Transgenic Nicotiana plumbaginifolia calli were homogenized, and MnSOD activity was assayed by in situ staining after electrophoresis in non-denaturing polyacrylamide gels, using homogenization and electrophoresis procedures as described in Example 2. The gels were treated with KCN and $H_2O_2$ prior to staining so that only the manganese isoforms of the enzyme were revealed. The results of these assays are presented in Figure 10 in which the MnSOD activity in control calli and calli transformed with pEX1SOD, pEX3SOD and pEX4SOD can be seen.

The endogenous MnSOD is clearly marked. The lane for pEX1SOD shows the 35S-mit-tp-MnSOD construction to be expressed and to be targeted to the mitochondria as the additional MnSOD comigrates with the endogenous enzyme. This results in a band of approximately double intensity when compared to the control.

The lane for pEX3SOD shows a faint band below the endogenous band. This band represents the MnSOD as expressed in the cytoplasm.

The lane for pEX4SOD shows two additional bands, one of which is at the same position as the faint band in the pEX3SOD lane. This represents the MnSOD expressed in the chloroplasts which suggests that the SSU transit peptide is cleaved off as expected. The other faint band in this lane, with an intermediate position with respect to the endogenous band and the processed form described above, may represent an unprocessed form.

The results of these MnSOD activity assays in transgenic calli prove that the recombinant SOD genes, which were introduced, were actively expressed. The expression level of both pEX1SOD and pEX4SOD is equivalent to that of the endogenous SOD gene as expressed in calli while the expression level of pEX3SOD is approximately one-tenth of that of the endogenous SOD gene. As the endogenous MnSOD is highly expressed in calli, the chimaeric constructions give similarly high expression.

Similar results were obtained for leaf tissue of transformed N. tabacum PBD6. Moreover, in PBD6 plants, the expression levels as determined by SOD activity staining were found to correspond well with mRNA levels determined by Northern blotting using the cDNA of the MnSOD as a probe and with protein levels determined either by sodium dodecyl sulphate ("SDS") polyacrylamide gel electrophoresis (Laemmli, 1970) or by Western blotting using polyclonal antisera raised against the MnSOD overexpressed in yeast (See Bowler et al., 1989b for expression of MnSOD in yeast).

To see whether the MnSOD expression was efficiently targeted to the desired cell compartments, leaf tissue of plants transformed with pEX1SOD, pEX3SOD and pEX4SOD was homogenized, and subcellular fractions were prepared. Fractions representing the chloroplast stroma and membranes were prepared according to Van den Broeck et al. (1985) and Mullet and Chua (1983), while fractions corresponding to the mitochondrial matrix and membranes were prepared according to Boutry et al. (1987). In addition, in situ immunolocalization of the MnSOD was performed on thin sections prepared from leaf tissue of transgenic plants (Greenwood and Chrispeels, 1985), using polyclonal antisera raised against the MnSOD (prepared as described above).

Both approaches showed that MnSOD activity in PBD6 plants transformed with pEX4SOD was exclusively localized in the chloroplasts, as expected. Total SOD activity in the chloroplasts of these plants was approximately doubled.

Mitochondrial targeting was also shown to be very effective in PBD6 tobacco plants transformed with pEX1SOD since MnSOD activity was exclusively localized within the mitochondria. Total SOD activity in these organelles was increased 20-fold with respect to nontransformed control plants.

Cytosolic expression was shown to be the least effective. Although total SOD activity in the cytosol of PBD6 plants transformed with pEX3SOD was not significantly increased as compared to control plants, the presence of MnSOD activity could be unequivocally determined while no increased MnSOD activity was found in the chloroplasts and mitochondria.

Example 10: Tolerance of transgenic plants to increased levels of superoxide radicals.

In order to observe the tolerance of the transgenic plants of Example 9 to increased levels of superoxide radicals, plants were subjected to various concentrations of methyl viologen ("MV"). This substance is known to generate superoxide radicals, especially in chloroplasts, and its effects have been well characterized. In general, superoxide radicals, generated by the action of MV, peroxidize the lipids in biological membranes, initiating a chain propagation reaction. In chloroplasts, this eventually leads to an oxidation of the photosynthetic pigments in the membranes (Halliwell, 1984). Experiments with MV therefore provide a convenient model system to study alterations in the physiological state of transgenic plants overexpressing the MnSOD. Results are expected to have a direct bearing on situations in which plants are subjected to natural, superoxide radical-producing, stress conditions.

In general, leaf pieces of transformed and untransformed plants were incubated in Petri dishes containing various amounts of MV. To stimulate electron transport, carbonylcyanide p-trifluoromethoxyphenylhydrane ("FCCP") was also included. After incubation, factors such as weight changes (due to membrane damage), pigment bleaching and the extent of lipid peroxidation were assayed and compared.

1. Weight Changes

Leaf discs of constant size (7x22 mm) and identical age were incubated in the light in aqueous solutions containing 2uM FCCP and various concentrations of MV for 21 hours at 20°C. The leaf discs were weighed before and after incubation. Percent weight change for two transgenic plants (T16-213 and T16-202) as compared to a control plant (T17-50) for different concentrations of MV is shown in Fig. 11. Each measurement was made for two separate leaf discs of the same plant and curves were drawn between the averages of the paired values.

The results clearly show the protective effect of chloroplastic MnSOD overexpression against loss of membrane integrity due to the action of MV.

2. Chlorophyll bleaching.

Incubation of leaf discs was performed as described above. After incubation, a pigment extract of the leaf discs was prepared as follows. Discs were homogenized in a mortar and pestle using $Al_2O_3$ as an abrasive. After addition of 3.1 ml of a chloroform/methanol/water mixture (1:2:0.1) and mixing, the suspension was allowed to stand in the dark for 5 minutes. The supernatant was transferred to a centrifuge tube, and 2.4 ml of a chloroform/methanol mixture (1:2) was added to the mortar. After a new extraction period of 5 minutes in the dark, the supernatant in the mortar was added to the centrifuge tube. The combined supernatants were then centrifuged for 15 minutes at 3300 rpm (Sorvall HB4 rotor) at room temperature (25° C). The extinction of the supernatant was measured at 664 nm ($E_{final}$). $E_{664}$ was also determined for pigment extracts of comparable leaf discs without incubation ($E_{ini}$). Fig. 12 shows "percent bleaching" [$\%E = 100x(E_{ini}-E_{fin})/E_{ini}$] as a function of the MV concentration.

To eliminate possible effects of differences in initial chlorophyll content of the discs derived from different plants, a second experiment was performed as follows. Leaf discs from different plants were incubated for one day in an aqueous solution containing 50 uM MV. Percentage bleaching was calculated and standardized with respect to the initial amounts of chlorophyll present in each plant. The results are shown in Fig. 13. It can be seen that %E decreases linearly with increasing initial chlorophyll content (as measured by $E_{ini}$ per 35 mg of chlorophyll) at least for control (T17-50 and PBD6), cytosolic MnSOD (T16-100 and T16-109) and mitochondrial MnSOD plants (T16-7 and T16-37). Chloroplastic MnSOD plants (T16-202 and T16-213), by comparison, are well below this line, indicating a protection against pigment oxidation in these plants.

3. Lipid peroxidation.

The assay for the extent of lipid peroxidation of polyunsaturated fatty acids is based on the detection of malondialdehyde, a decomposition product of lipid peroxides. Production of malondialdehyde is enhanced by acidic conditions. Malondialdehyde reacts with thiobarbituric acid ("TBA") to produce a red chromogen which can be measured photometrically at 532 nm (Slater, 1984). The increase in TBA reactivity ("TBAR") is thus a direct measure of lipid peroxidation.

PBD6 leaf discs (0.4 g) of approximately 1 $cm^2$ were incubated in the light at room temperature in petri dishes containing 10 ml 50 mM Tris/HCl, pH 7.0, with different concentrations of MV (0, 0.1, 0.5 and 1.0 mM). After a two hour incubation period, tissue was homogenized in: 3.5 ml 6mM $NaH_2PO_4$; 1.2 mg/l ethylenediamine tetraacetate ("EDTA"); and 0.265% (w/v) TBA in 0.17 M HCl. The homogenate was placed in a boiling water bath for 15 minutes, cooled to room temperature and centrifuged at 12000 rpm for 10 minutes (Eppendorf centrifuge). The absorbance of the supernatant was read at 532 nm and converted to the amount of malondialdehyde per gram of fresh tissue.

TBAR for all transgenic plants was less than for control PBD6 plants (Figure 14A). Chloroplastic MnSOD plants are clearly best protected against lipid peroxidation, and this effect could be shown to be dependent upon the expression level of the MnSOD (see Fig. 14B).

Example 11: Tolerance of transgenic plants to stress conditions.

The response of the transgenic plants of Example 9 to different stress situations was analyzed in depth through monitoring of fitness components such as growth and survival characteristics. Comparison is made to

suitable controls (i.e., non-transformed plants). Environmental differences and/or differences in genetic background among the tested plants necessitate a statistical approach.

In one case, the germination of seeds of Arabidopsis plants transformed with pEX1SOD, pEX3SOD and pEX4SOD was assayed under conditions of constant light. Seeds of transformed and untransformed plants were germinated on K1 medium (Valvekens et al., 1988) in petri dishes sealed with two layers of Urgopore(R) porous tape (from Chenove Co. in France) and placed under conditions where the light intensity was held constant at 2500 lux. The temperature of the growth chamber was set at 21°C. The experiment was initiated on January 26, 1989 (winter), and due to the configuration of the growth chamber, chamber temperature was somewhat influenced by outside temperatures and fluctuated between 10° and 20°C in phase with the outside temperatures.

For each tested Arabidopsis line, three K1 plates, with 100 seeds each, were placed in the chamber. Plates were randomly spaced in sets of three. After 15 days, the condition of the seedlings was assayed as follows: 0, not germinated; 1, germinated but seedlings show obvious signs of bleaching and/or retardation (indicative of environmental stress); and 2, germinated to normal seedlings. Further analyses were only performed for germinated seeds. Table 1 below displays, for each plant, the total number of seedlings scored in the two classes. For each plant, the value of chi square ("$X^2$" -- Snedecor and Cochran, 1980) was calculated with respect to the results from the control plant (nontransformed Arabidopsis). From Table 1, it can be seen that all transgenic plants (i.e., 1SOD-, 3SOD- and 4SOD-plants transformed with pEX1SOD, pEX3SOD and pEX4SOD, respectively) have more normal seedlings (class 2) than the control plant. In all cases, the differences are highly significant.

Table 1

| Arabidopsis line | Class 1 seedlings | Class 2 seedlings | $X^2$ |
|---|---|---|---|
| C24 (control) | 151 | 18 | |
| 1SOD4 | 54 | 71 | 72.5 |
| 1SOD5 | 52 | 175 | 194.7 |
| 1SOD7 | 38 | 204 | 241.9 |
| 3SOD5 | 106 | 49 | 37.3 |
| 3SOD11 | 103 | 76 | 80.5 |
| 4SOD1 | 58 | 186 | 252.6 |
| 4SOD7 | 65 | 124 | 112.6 |
| 4SOD2 | 65 | 122 | 110.9 |

Surface sterilized seeds of Arabidopsis thaliana (both untransformed and transformed with pEX1SOD, pEX3SOD or pEX4SOD) are germinated on germination medium as described by Valvekens et al. (1988). In the case of transgenic plants, the medium is supplemented with 50 mg/l kanamycin sulphate. One week old seedlings are transferred to germination medium supplemented with Fe(II)-EDTA at concentrations of 500 uM, 250 uM and 100 uM. The plants are grown according to Valvekens et al. (1988), and the tolerance of transformed plants to the toxic effects of Fe (II) appears to be better than the tolerance of untransformed (wild-type) plants.

Example 12: Isolation of a cDNA clone from Nicotiana plumbaginifolia encoding an FeSOD.

cDNAs prepared from mRNAs, derived from a suspension culture of Nicotiana plumbaginifolia (see Example 1), were cloned in the PstI site of plasmid pUC18 (Yannisch-Perron et al., 1985) by means of homopolymeric dG-dC tailing as follows. pUC18 was linearized with PstI and a dG tail was added to the 3′ ends by means of terminal deoxynucleotidyl transferase. Complementary dC-tails were added to the 3′ ends of the cDNAs. 1 ug of the resulting cDNA library was electroporated in $10^9$ cells of an E.coli strain sodAsodB$^-$ (Carlioz and Touati, 1986) by means of the Biorad Gene Pulser (BioRad Chemical Division, 1414 Harbour Way, South Richmond, California 94804, U.S.A.) according to the procedure described in the BioRad manual. E.coli sodAsodB$^-$ is deficient in SOD activity and is unable to grow aerobically on minimal medium. Consequently, cDNA clones encoding SOD enzymes can be isolated by growing transformants on minimal medium. After 3 days of incubation at 37°C, a total of 67 colonies could be picked up. The inserts of some of the clones did not hybridize with the cDNA coding for the MnSOD of Example 1. Restriction analysis of these clones showed that many contained very similar inserts. Proteins were extracted from colonies containing these plasmids, and assays for SOD activity on polyacrylamide gels (Beauchamp and Fridovich, 1971) confirmed that these colonies synthesized a protein with SOD activity. Inhibition studies (Bowler et al., 1989 a) further showed the SOD to be resistant to KCN and sensitive to $H_2O_2$, results which are indicative of an FeSOD (Bannister et al., 1987). The pUC18 insert was sequenced (Sanger et al., 1977), and the DNA sequence is shown in Fig. 5. This is the first cDNA of an FeSOD to be isolated from an eucaryote. The first in frame codon after the dC-dG tail is a lysine, indicating that transcription is initiated from the lacZ-ATG codon of pUC18 and that the FeSOD is

synthesized as a fusion protein. The FeSOD in leaf tissue of Nicotiana plumbaginifolia was found to be localized within the chloroplasts and to be highly responsive to stress conditions like MV, heat shock, sucrose, and infection by Pseudomonas syringae.

Plant expression vectors containing the FeSOD gene are prepared using the procedure of Examples 3-6, and such vectors are used to transform the same plant species as in Example 8, using the general procedures of Example 8, whereby the plants express the FeSOD gene.

Needless to say, this invention is not limited to the transformation of any specific plant(s). The invention relates to any plant, the genome of which can be transformed with an SOD gene, particularly a plant MnSOD or FeSOD gene, that is under the control of a promoter capable of directing expression of the SOD gene in the plant's cells and that is preferably fused at its 5′ end to a targeting sequence encoding a targeting peptide for translocation within, or secretion from, the cells of the plant of an expressed SOD, to provide the plant with increased resistance and/or tolerance to a naturally occurring stress condition which produces toxic, highly reactive, oxygen species in one or more of the plant cell compartments.

This invention also is not limited to the plant SOD genes of Figures 1, 2, 3 and 5, used in the foregoing Examples. In this regard, the invention encompasses MnSOD and FeSOD genes encoded by: 1) any DNA fragments differing from the SOD genes of Figures 1, 2, 3 and 5 by the replacement of any of their nucleotides by others, without modifying their genetic information (normally within the meaning of the universal genetic code); and 2) any DNA fragments that encode polypeptides which have the same or equivalent SOD properties as the polypeptides, encoded by the SOD genes of Figures 1, 2, 3 and 5, but which may not have the same amino acid residues. Likewise, this invention is not limited to the MnSODs and FeSODs of Figures 1, 2, 3 and 5 but rather covers any equivalent polypeptides. Indeed, it is apparent that one skilled in the art will be able to remove 5′ and/or 3′ portions of the SOD genes of Figure 1, 2, 3 and 5 without significantly affecting their usefulness for transforming plants to render them stress resistant in accordance with this invention. Such portions may be removed, for example, by removing terminal parts on either side of a SOD gene with an exonucleolytic enzyme (e.g., Bal31), and the remaining shortened DNA fragment can then be recovered in a suitable plasmid so that the capacity of the modified plasmid to transform plant cells and to enhance SOD production therein (e.g., as measured by the assay described in Example 2) can be determined. Such a shortened DNA fragment, coding for a shortened SOD which retains its SOD activity, is considered an equivalent of an SOD gene of Figure 1, 2, 3 or 5. Likewise, such a shortened SOD is considered an equivalent of an SOD of Figure 1, 2, 3 or 5.

Furthermore, this invention is not limited to the promoters, 3′ transcription regulation signals and targeting sequences used in the Examples. One skilled in the art will be able readily to substitute different DNA fragments and regulatory sequences which can perform equivalent functions in the recombinant SOD gene of this invention in a transformed plant cell, cell culture or plant.

Also, this invention is not limited to the specific plasmids and vectors described in the foregoing Examples, but rather encompasses any plasmids and vectors containing the recombinant SOD gene of this invention, useful for obtaining expression of the SOD gene in one or more plant cell compartments.

References

- ARRIGONI, ZACHEO, BLEVE-ZACHEO, ARRIGONI-LISO and LAMBERTI, 1981, Nematol. Medit. 9:195-198
- ASADA, KISO and YOSHIKAWA, 1974, J. Biol. Chem. 247:2175-2181
- BANNISTER, BANNISTER and ROTILIO, 1987, CRC Crit. Rev. Biochem. 22:111-180
- BAUW, DE LOOSE, INZE, VAN MONTAGU and VANDEKERCKHOVE, 1987, PNAS USA* 84:4806-4810 (* Proceedings of the National Academy of Sciences, USA)
- BARRA, SCHININA, SIMMACO, BANNISTER, BANNISTER, ROTILIO and BOSSA, 1984, J. Biol. Chem. 259:12595-12601
- BEAUCHAMP and FRIDOVICH, 1971, Anal. Biochem. 44:276-287
- BEEVERS, 1979, Ann. Rev. Plant Physiol. 30:159-193
- BERMINGHAM-McDONOGH, GRALLA and VALENTINE, 1988, PNAS USA 85:4789-4793.
- BOUTRY, NAGY, POULSEN, AOYAGI and CHUA, 1987, Nature 328:340-342
- BOWLER, ALLIOTTE, DE LOOSE, VAN MONTAGU and INZE, 1989a, EMBO J. 8:31-38
- BOWLER, ALLIOTTE, VAN DEN BULCKE, AUW, VANDEKERCKHOVE, VAN MONTAGU and INZE, 1989b, PNAS USA 3237-3241
- BRIDGES and SALIN, 1981, Plant Physiol. 68:275-278
- BROCK and WALKER, 1980, Biochemistry 19:2873-2882
- BROWN and FRIDOVICH, 1981, Arch. Biochem. Biophys. 206:414-419
- CANNON, WHITE and SCANDALIOS, 1987, PNAS USA 84: 179-183
- CARLIOZ LUDWIG, STALLINGS, FEE, STEINMAN, TOUATI, 1988, J. Biol. Chem. 263:1555-1562
- CARLIOZ and TOUATI, 1986, EMBO J. 5:623-630
- CASHMORE, 1983, in "Genetic Engineering of Plants" (Kosugen, Meredith and Hollaender, eds.) Plenum Press.
- CLARE, RABINOWITCH and FRIDOVICH, 1984, Arch. Biochem. Biophys. 231:158-163
- COLLINGE and SLUSARENKO, 1987, Plant. Mol. Biol.9:389-410

- DAUB, 1982, Plant Physiol. 69:1361-1364
- DAUB, 1987, Phytopathology 77:1515-1520
- DAUB and BRIGGS, 1983, Plant Physiol. 71:763-766
- DAUB and HANGARTER, 1983, Plant Physiol. 73:855-857
- DEBLAERE et al., 1985, NAR* 13:4777-4788
(* Nucleic Acids Research)
- DEBLOCK et al., 1987, EMBO J. 6:2513-2518
- ELLIS et al., 1988, Plant Molecular Biol. 10:203-214
- FRANCK, GUILLEY, JONARD, RICHARDS and HIRTH, 1980, Cell 21:285-294
- FRIDOVICH, 1987, Science 201:875-880
- FUCCI, OLIVER, COON and STADTMAN, 1983, PNAS USA 80:1521-1525
- FURUSAWA and MIZUGUCHI, 1988, 5th Intl. Cong. of Plant Pathology, Aug. 20-24, 1988, Kyoto, Japan, Abstracts of Papers 2.1.
- FURASAWA, TANAKA, THANUTONG, MIZUGUCHI, YAZAKI and ASADA, 1984, Plant Cell Physiol. 25:1247-1254
- GARDNER, HOWARTH, HAHN, BROWN-LUEDI, SHEPARD and MESSING, 1981,NAR 9:2871-2887
- GETZOFF, TAINER, STEMPIEN, BELL and HALLEWELL, 1989, Proteins, 5:322-336
- GIELEN, DE BEUCKELEER, SEURINCK, DEBOECK, DE GREVE, LEMMERS, VAN MONTAGU and SCHELL, 1984, EMBO J. 3:835-845
- GREENWOOD and CHRISPEELS (1985) Plant Physiol. 79:65-71
- GRIMES, PERKINS and BOSS, 1983, Plant Physiol. 72:1016-1020
- GUBLER and HOFFMAN, 1983, Gene 25:263-269.
- HAIN, STABEL, CZERNILOVSKY, STEINBISS, HERRERA-ESTRELLA and SCHELL, 1985, Mol. Gen. Genet. 199:161-168
- HALLIWELL, 1984, "Chloroplast Metabolism", Clarendon Press, Oxford
- HARBOUR and BOLTON, 1975, Biochem. Biophys. Res. Commun. 64:803-807
- HARPSTER, TOWNSEND, JONES, BEDBROOK and DUNSMUIR, 1988, Mol. Gen. Genet. 212:182-190
- HARRIS and STEINMAN, 1977, in "Superoxide and superoxide dismutase", Michelsen, McCord and Fridovich (eds.), Academic Press, London, p225-230
- HARTMAN, SUZUKI and STACK, 1989, Appl.Environmental Microbiol. 1989:7-14
- HASSAN and FRIDOVICH, 1979, Arch. Biochem. Biophys. 196:385-395
- HO and CRAPO, 1988, FEBS 229:256-260
- HOHN and SCHELL, 1987 (eds.) Plant Infectious Agents. Springer Verlag Wien, New York.
- HULL and HOWELL, 1978, Virology 86:482-493
- IMLAY and LINN, 1988, Science 240:1302-1309
- JONES, DUNSMUIR and BEDBROOK, 1985, EMBO J. 4:2411-2418
- KAISER, 1979, Planta 145:377-382
- KUHLEMEIER, GREEN and CHUA, 1987, Ann. Rev. Plant. Physiol. 38:221-257
- LAEMMLI, 1970, Nature, 227:680-685
- LEE and BENNETT, 1982, Plant Physiol. 69:1444-1449
- LEE, BOCHNER and AMES, 1983, PNAS USA 80:7496-7500
- MACKAY, SENARATNA, MCKERSIE and FLETCHER, 1987, Plant Cell Physiol. 28:1271-1278
- MANIATIS, FRITSCH and SAMBROOK, 1982, Molecular Cloning A laboratory Manual, Cold Spring Harbor Laboratory, Cold Spring Harbor
- MARRES, VAN LOON, OUDSHOORN, VAN STEEG, GRIVELL and SLATER, 1985, Eur. J. Biochem. 147:153-161.
- MATTERS and SCANDALIOS, 1986, Biochem. Biophys. Acta 882:29-38
- MAXAM and GILBERT, 1980, Methods Enzymol. 65(1):499-559, Academic Press
- MEAD, 1976, in "Free Radicals in Biology Vol 1' (Pryor, ed) pp. 51-68 Academic Press
- MONK, FAGERSTEDT and CRAWFORD, 1987, Plant Physiol. 85:1016-1020
- MULLET and CHUA, 1983, Meth.Enzymol. 97:502-509
- MUNKRES, RANA and GOLDSTEIN, 1984, Mechanisms of Ageing and Development 24:83-100
- MURASHIGE and SKOOG, 1962, Physiol. Plantarum 15:473-497
- NAGY and MALIGA, 1976, Z. Pflanzenphysiol. 78:453-455
- ODELL et al., 1983, Nature 313:810-812
- OHTSUKA, MATSUKI, IKEHARA, TAKAHSHI, MATSUBARA, 1985, J. Biol. Chem. 260:2605-2608
- ORR and HOGAN, 1983, Pestc. Biochem. Biophys. 20:311-319
- PARKER and BLAKE, 1988, FEBS Letters 229:377-382
- PENG, MAYA and AYALA, 1986, PNAS USA 83:684-687
- POWLES (1984) Ann. Rev. Plant Physiol. 35: 15-44
- RABINOWITCH and FRIDOVICH, 1985, PLANTA 164: 524-528
- RABINOWITCH, SKLON and BUDOROSKI, 1982, Physiol. Plant. 54:369-374
- REISS, SPRENGEL and SCHALLER, 1984, EMBO J. 3:3317-3322
- ROBINSON, SMITH and GILLS, 1980, Plant Physiol. 65:755-759
- SACHS and HO, 1986, Ann. Rev. Plant. Physiol. 65:755-759

- SCHATZ, 1987, Eur. J. Biochem. 165:1-6
- SETO, HAYASHI and TENER, 1987, NAR 15:10601
- SHAALTIEL and GRESSEL, 1987, Plant Physiol. 85:869-871
- SIMOENS et al., 1986, NAR 14:8073-8090
- SINGLETON and SAINSBURY, 1987, Dictionary of Microbiology and Molecular Biology, Wiley and Sons
- SLATER, 1984, Meth.Enzymol. 105:283-293
- SNEDECOR and COCHRAN, 1980, "Statistical Methods", Iowa State University Press.
- SOBEL and MARTINEZ, 1986, NAR 14:363-374
- STALLINGS, PATTRIDGE, STRONG and LUDWIG, 1985, J. Biol. Chem. 260:16424-16432
- STANSSENS, McKEOWN, FRIEDRICH and FRITZ, 1987, Manual EMBO Laboratory course "Directed mutagenesis and protein engineering" held at Max Planck Institute für Biochemie, Martinsried, West-Germany, July 4-18, 1987.
- STEINITZ et al, 1979, Plant Science Letters p327-335
- STEINMAN, 1978, J. Biol. Chem. 253:8708-8720
- TAKAHASHI et al., 1985, PNAS USA 82:1931-1935
- TANAKA and SUGAHARA, 1980, Plant Cell Physiol. 21:601-612
- TANAKA, KONDO and SUGAHARA, 1982, Plant Cell Physiol. 23:999-1007
- VALVEKENS, VAN MONTAGU and VAN LYSEBETTENS, 1988, PNAS USA 85:5536-5540.
- VAN DEN BROECK, TIMKO, KAUSCH, CASHMORE, VAN MONTAGU and HERRERA-ESTRELLA, 1985, Nature, 358-363
- VELTEN and SCHELL, 1985, NAR 13:6998
- VELTEN, VELTEN, HAIN and SCHELL, 1984, EMBO J. 3:2723-2730
- VON HEIJNE, 1988, Bioch. Biophys. Acta 947: 307-333
- WATSON, 1984, NAR 12:5145-5164
- WINNACKER, 1987, From Genes to Clones, VCH Verlagsgesellschaft
- WU and GROSSMAN (eds.), 1987, "Methods in Enzymology", Vol. 153 Part D, Academic Press.
- YANNISH-PERRON, VIERA and MESSING, 1985, Gene 33:103-119
- ZACHEO, BLEVE-ZACHEO and LAMBERTI, 1982, Nemat. Medit. 10:75-80

## Claims

1. A recombinant SOD gene, preferably a chimaeric recombinant SOD gene, for transforming a plant cell, characterized by the following DNA fragments which are operably linked and in the same transcriptional unit:

a) an SOD gene encoding a metallo-superoxide dismutase, particularly a plant superoxide dismutase, quite particularly a Mn or Fe superoxide dismutase;

b) a promoter capable of directing expression, preferably overexpression, of said SOD gene in said plant cell; and

c) 3′ transcription regulation signals for expression of said SOD gene in said plant cell.

2. The recombinant SOD gene of claim 1, further characterized by a targeting sequence which encodes a targeting peptide and which is located between said promoter and said SOD gene and is fused to, and in the same reading frame as, said SOD gene; said targeting peptide being adapted for translocation of said superoxide dismutase into mitochondria or chloroplasts of said cell or into the lumen of the endoplasmatic reticulum of said plant cell for secretion of said superoxide dismutase out of said plant cell.

3. The recombinant SOD gene of claim 1 or 2 wherein said SOD gene is a plant MnSOD gene or FeSOD gene, particularly a gene having the DNA sequence shown in Figure 1, 2, 3 or 5.

4. A plant cell, the genome, particularly the nuclear genome, of which is transformed with the recombinant SOD gene of anyone of claims 1-3.

5. A plant consisting of the plant cells of claim 4.

6. A plant cell culture consisting of the plant cells of claim 4.

7. A seed of the plant of claim 5.

8. Plant expression vectors characterized by the recombinant SOD gene of anyone of claims 1-3 between the border sequences of a disarmed T-DNA of Agrobacterium, particularly pEX1SOD, pEX3SOD, pEX4SOD, pEX5SOD, pEX6SOD, pEX7SOD or pEX8SOD.

9. A plant FeSOD gene or MnSOD gene, particularly a gene having the DNA sequence shown in Figure 1, 2, 3 or 5.

10. A plant Fe superoxide dismutase or Mn superoxide dismutase, particularly a superoxide dismutase having the amino acid sequence shown in Figure 1, 2, 3 or 5.

11. In a process for producing plant and reproductive material, such as seeds, or for producing fruits of said plants including a foreign genetic material stably integrated in nuclear genome thereof and capable of being expressed therein as an RNA, protein or polypeptide, comprising the non-biological steps of: a) producing transformed plants cells or plant tissue including said foreign genetic material from starting plant cells or plant tissue not expressing said RNA, protein or polypeptide, b) producing regenerated

plants or reproductive material of said plants or both from said transformed plant cells or plant tissue including said foreign genetic material, and c) optionally, biologically replicating said regenerated plants or reproductive material or both; wherein said step of producing said transformed plant cells or plant tissue including said foreign genetic material is characterized by: transforming the nuclear genome of said starting plant cells or plant tissue with a recombinant SOD gene of anyone of claims 1-3, as well as regulatory elements which are capable of enabling the expression of said foreign DNA sequence in said plant cells or plant tissue, to cause the stable integration of said foreign DNA sequence in transformed plant cells or plant tissue, as well as in plants and reproductive material produced therefrom throughout subsequent generations.

12. A method for rendering a plant more resistant or tolerant to toxic, highly reactive, oxygen species, particularly superoxide anions, produced in the plant's cells as a result of a stress condition, particularly a naturally occurring stress condition, characterized by the step of: transforming the genome, preferably the nuclear genome, of said plant with the recombinant SOD gene of anyone of claims 1-3, whereby an SOD is produced, preferably overproduced, in cells of said plant.

Figure 1

```
          GGGGGGGGGG GGGGGGCTGG CCTCTCTGGG CATGACCTGC AACTATAAAA GGACACCATA

GAGTTAACAG CTAGAAAGCA TTTAGGAATA TCTCAAAA

                                 27                                  54
ATG GCA CTA CGA ACC CTA GTG AGC AGA CGG ACC TTA GCA ACA GGG CTA GGG TTC
MET Ala Leu Arg Thr Leu Val Ser Arg Arg Thr Leu Ala Thr Gly Leu Gly Phe

                         ↓       81                                 108
CGC CAG CAA CTC CGC GGC TTG CAG ACC TTT TCG CTC CCC GAT CTC CCC TAC GAC
Arg Gln Gln Leu Arg Gly Leu Gln Thr Phe Ser Leu Pro Asp Leu Pro Tyr Asp

                                135                                 162
TAT GGA GCA CTG GAG CCG GCA ATT AGC GGT GAC ATA ATG CAG CTC CAC CAC CAG
Tyr Gly Ala Leu Glu Pro Ala Ile Ser Gly Asp Ile MET Gln Leu His His Gln

                                189                                 216
AAT CAC CAT CAG ACT TAC GTC ACC AAT TAC AAT AAA GCC CTT GAA CAG CTA CAT
Asn His His Gln Thr Tyr Val Thr Asn Tyr Asn Lys Ala Leu Glu Gln Leu His

                                243                                 270
GAC GCC ATT TCC AAA GGA GAT GCT CCT ACC GTC GCC AAA TTG CAT AGC GCT ATC
Asp Ala Ile Ser Lys Gly Asp Ala Pro Thr Val Ala Lys Leu His Ser Ala Ile
```

Figure 1 (cont. 1)

```
                                297                                                  324
AAA TTC AAC GGC GGA GGT CAC ATT AAC CAC TCG ATT TTC TGG AAG AAT CTT GCC
Lys Phe Asn Gly Gly Gly His Ile Asn His Ser Ile Phe Trp Lys Asn Leu Ala

                                351                                                  378
CCT GTC CGC GAG GGT GGT GGT GAG CCT CCA AAG GGT TCT CTT GGT TGG GCT ATC
Pro Val Arg Glu Gly Gly Gly Glu Pro Pro Lys Gly Ser Leu Gly Trp Ala Ile

                                405                                                  432
GAC ACT AAC TTT GGC TCC CTA GAA GCT TTA GTT CAA AAG ATG AAT GCA GAA GGT
Asp Thr Asn Phe Gly Ser Leu Glu Ala Leu Val Gln Lys MET Asn Ala Glu Gly

                                459                                                  486
GCT GCT TTA CAG GGC TCT GGC TGG GTG TGG CTT GGT GTG GAC AAA GAG CTT AAG
Ala Ala Leu Gln Gly Ser Gly Trp Val Trp Leu Gly Val Asp Lys Glu Leu Lys

                                513                                                  540
CGC CTG GTG ATT GAA ACC ACT GCT AAT CAG GAC CCT TTG GTT TCT AAA GGA GCA
Arg Leu Val Ile Glu Thr Thr Ala Asn Gln Asp Pro Leu Val Ser Lys Gly Ala

                                567                                                  594
AAT TTG GTT CCT CTT CTG GGA ATA GAC GTT TGG GAA CAT GCA TAC TAC TTG CAG
Asn Leu Val Pro Leu Leu Gly Ile Asp Val Trp Glu His Ala Tyr Tyr Leu Gln
```

Figure 1 (cont. 2)

```
                                621                                                  648
TAC AAA AAT GTA AGA CCT GAT TAT CTG AAG AAC ATA TGG AAA GTT ATG AAC TGG
Tyr Lys Asn Val Arg Pro Asp Tyr Leu Lys Asn Ile Trp Lys Val MET Asn Trp

                                675                 *
AAA TAT GCA AAT GAA GTT TAT GAG AAA GAA TGT CCT TGAACAGGGA TATTTGATGT
Lys Tyr Ala Asn Glu Val Tyr Glu Lys Glu Cys Pro  .

TGTTTTGAGG ACGTCTGTAA AACTTTTTGA TGGGAAATAA GGCTGAGTGA CATGAGCAGG

TGTCCTGTTT TTCTTGCATG TAGTCGCTGG CTGATGTACT TGATGTATTT CTGGAAAAGG

TTGATGTATG TACTTGATAT ATGGAGCCTA AATAAAACTA CTCTATCGTT TGAGCGCAAA

CCCCCCCCCC CCCC
```

Figure 2

```
                    ↓                       27                                  54
ATG GAT CTG GGC TTG CAG ACC TTT TCG CTC CCC GAT CTC CCC TAC GAC TAT GGA
MET Asp Leu Gly Leu Gln Thr Phe Ser Leu Pro Asp Leu Pro Tyr Asp Tyr Gly

                                     81                                 108
GCA CTG GAG CCG GCA ATT AGC GGT GAC ATA ATG CAG CTC CAC CAC CAG AAT CAC
Ala Leu Glu Pro Ala Ile Ser Gly Asp Ile MET Gln Leu His His Gln Asn His

                                    135                                 162
CAT CAG ACT TAC GTC ACC AAT TAC AAT AAA GCC CTT GAA CAG CTA CAT GAC GCC
His Gln Thr Tyr Val Thr Asn Tyr Asn Lys Ala Leu Glu Gln Leu His Asp Ala

                                    189                                 216
ATT TCC AAA GGA GAT GCT CCT ACC GTC GCC AAA TTG CAT AGC GCT ATC AAA TTC
Ile Ser Lys Gly Asp Ala Pro Thr Val Ala Lys Leu His Ser Ala Ile Lys Phe

                                    243                                 270
AAC GGC GGA GGT CAC ATT AAC CAC TCG ATT TTC TGG AAG AAT CTT GCC CCT GTC
Asn Gly Gly Gly His Ile Asn His Ser Ile Phe Trp Lys Asn Leu Ala Pro Val

                                    297                                 324
CGC GAG GGT GGT GGT GAG CCT CCA AAG GGT TCT CTT GGT TGG GCT ATC GAC ACT
Arg Glu Gly Gly Gly Glu Pro Pro Lys Gly Ser Leu Gly Trp Ala Ile Asp Thr
```

Figure 2 (cont.)

```
                                351                                                378
AAC TTT GGC TCC CTA GAA GCT TTA GTT CAA AAG ATG AAT GCA GAA GGT GCT GCT
Asn Phe Gly Ser Leu Glu Ala Leu Val Gln Lys MET Asn Ala Glu Gly Ala Ala

                                405                                                432
TTA CAG GGC TCT GGC TGG GTG TGG CTT GGT GTG GAC AAA GAG CTT AAG CGC CTG
Leu Gln Gly Ser Gly Trp Val Trp Leu Gly Val Asp Lys Glu Leu Lys Arg Leu

                                459                                                486
GTG ATT GAA ACC ACT GCT AAT CAG GAC CCT TTG GTT TCT AAA GGA GCA AAT TTG
Val Ile Glu Thr Thr Ala Asn Gln Asp Pro Leu Val Ser Lys Gly Ala Asn Leu

                                513                                                540
GTT CCT CTT CTG GGA ATA GAC GTT TGG GAA CAT GCA TAC TAC TTG CAG TAC AAA
Val Pro Leu Leu Gly Ile Asp Val Trp Glu His Ala Tyr Tyr Leu Gln Tyr Lys

                                567                                                594
AAT GTA AGA CCT GAT TAT CTG AAG AAC ATA TGG AAA GTT ATG AAC TGG AAA TAT
Asn Val Arg Pro Asp Tyr Leu Lys Asn Ile Trp Lys Val MET Asn Trp Lys Tyr

                                621
GCA AAT GAA GTT TAT GAG AAA GAA TGT CCT TGA
Ala Asn Glu Val Tyr Glu Lys Glu Cys Pro  .
```

# Figure 3

```
                                    27                                          54
ATG GCT TCT ATG ATA TCC TCT TCC GCT GTG ACA ACA GTC AGC CGT GCC TCT AGG
MET Ala Ser MET Ile Ser Ser Ser Ala Val Thr Thr Val Ser Arg Ala Ser Arg

                                    81                                         108
GGG CAA TCC GCC GCA GTG GCT CCA TTC GGC GGC CTC AAA TCC ATG ACT GGA TTC
Gly Gln Ser Ala Ala Val Ala Pro Phe Gly Gly Leu Lys Ser MET Thr Gly Phe

                                   135                                         162
CCA GTG AAG AAG GTC AAC ACT GAC ATT ACT TCC ATT ACA AGC AAT GGT GGA AGA
Pro Val Lys Lys Val Asn Thr Asp Ile Thr Ser Ile Thr Ser Asn Gly Gly Arg

           ⇓                ↓  189                                         216
GTA AAG TGC ATG GAT CTG GGC TTG CAG ACC TTT TCG CTC CCC GAT CTC CCC TAC
Val Lys Cys MET Asp Leu Gly Leu Gln Thr Phe Ser Leu Pro Asp Leu Pro Tyr

                                   243                                         270
GAC TAT GGA GCA CTG GAG CCG GCA ATT AGC GGT GAC ATA ATG CAG CTC CAC CAC
Asp Tyr Gly Ala Leu Glu Pro Ala Ile Ser Gly Asp Ile MET Gln Leu His His

                                   297                                         324
CAG AAT CAC CAT CAG ACT TAC GTC ACC AAT TAC AAT AAA GCC CTT GAA CAG CTA
Gln Asn His His Gln Thr Tyr Val Thr Asn Tyr Asn Lys Ala Leu Glu Gln Leu
```

Figure 3 (cont. 1)

```
                                351                                             378
CAT GAC GCC ATT TCC AAA GGA GAT GCT CCT ACC GTC GCC AAA TTG CAT AGC GCT
His Asp Ala Ile Ser Lys Gly Asp Ala Pro Thr Val Ala Lys Leu His Ser Ala

                                405                                             432
ATC AAA TTC AAC GGC GGA GGT CAC ATT AAC CAC TCG ATT TTC TGG AAG AAT CTT
Ile Lys Phe Asn Gly Gly Gly His Ile Asn His Ser Ile Phe Trp Lys Asn Leu

                                459                                             486
GCC CCT GTC CGC GAG GGT GGT GGT GAG CCT CCA AAG GGT TCT CTT GGT TGG GCT
Ala Pro Val Arg Glu Gly Gly Gly Glu Pro Pro Lys Gly Ser Leu Gly Trp Ala

                                513                                             540
ATC GAC ACT AAC TTT GGC TCC CTA GAA GCT TTA GTT CAA AAG ATG AAT GCA GAA
Ile Asp Thr Asn Phe Gly Ser Leu Glu Ala Leu Val Gln Lys MET Asn Ala Glu

                                567                                             594
GGT GCT GCT TTA CAG GGC TCT GGC TGG GTG TGG CTT GGT GTG GAC AAA GAG CTT
Gly Ala Ala Leu Gln Gly Ser Gly Trp Val Trp Leu Gly Val Asp Lys Glu Leu

                                621                                             648
AAG CGC CTG GTG ATT GAA ACC ACT GCT AAT CAG GAC CCT TTG GTT TCT AAA GGA
Lys Arg Leu Val Ile Glu Thr Thr Ala Asn Gln Asp Pro Leu Val Ser Lys Gly
```

Figure 3 (cont. 2)

```
                                675                                             702
GCA AAT TTG GTT CCT CTT CTG GGA ATA GAC GTT TGG GAA CAT GCA TAC TAC TTG
Ala Asn Leu Val Pro Leu Leu Gly Ile Asp Val Trp Glu His Ala Tyr Tyr Leu

                                729                                             756
CAG TAC AAA AAT GTA AGA CCT GAT TAT CTG AAG AAC ATA TGG AAA GTT ATG AAC
Gln Tyr Lys Asn Val Arg Pro Asp Tyr Leu Lys Asn Ile Trp Lys Val MET Asn

                                783
TGG AAA TAT GCA AAT GAA GTT TAT GAG AAA GAA TGT CCT TGA
Trp Lys Tyr Ala Asn Glu Val Tyr Glu Lys Glu Cys Pro  .
```

Fig. 4 A

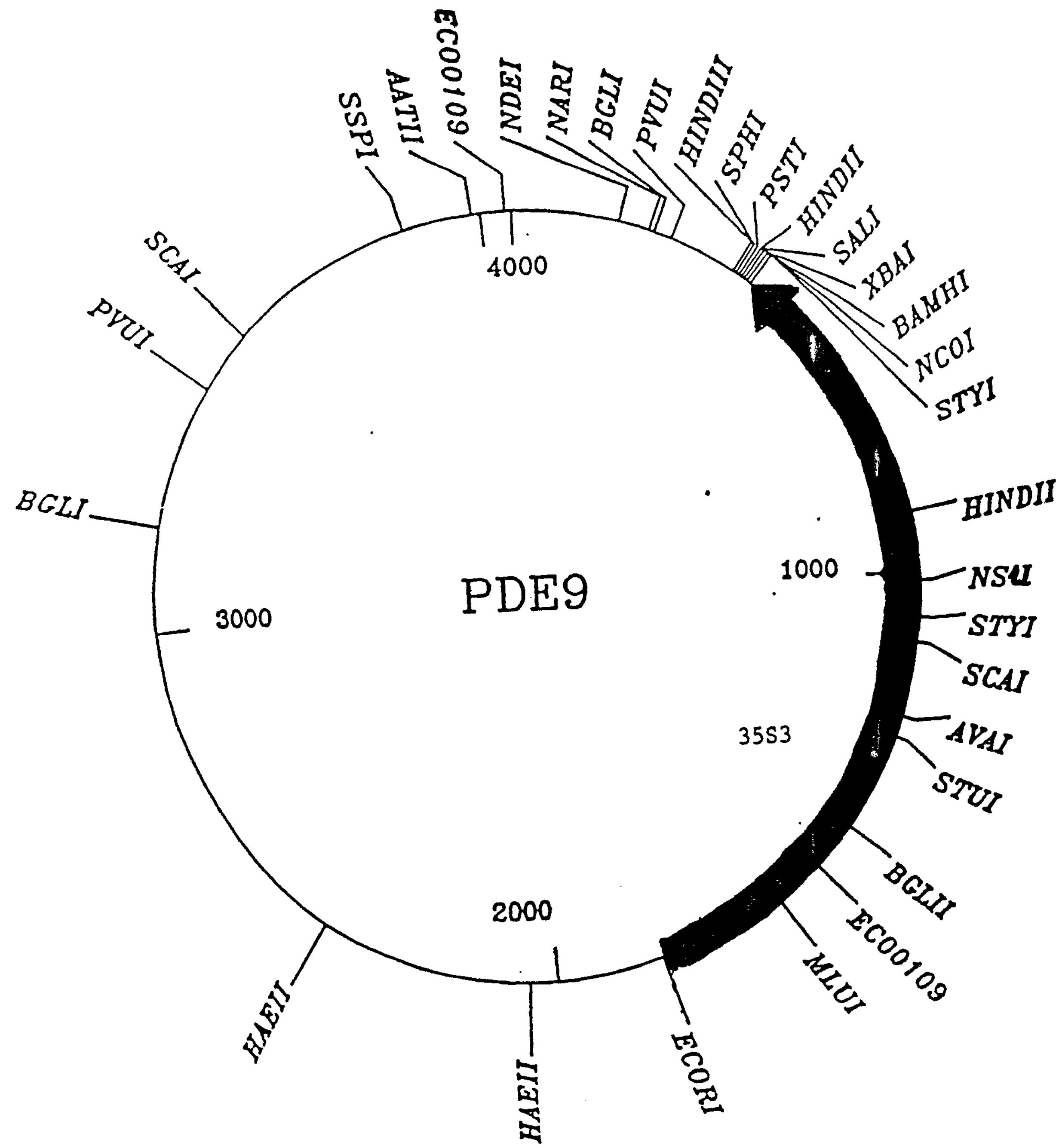
PDE9
SSPI
AATII
ECO0109
NDEI
NARI
BGLI
PVUI
HINDIII
SPHI
PSTI
HINDII
SALI
XBAI
BAMHI
NCOI
STYI
HINDII
1000
NSIL
STYI
SCAI
AVAI
STUI
35S3
BGLII
ECO0109
MLUI
ECORI
2000
HAEII
HAEII
3000
BGLI
PVUI
SCAI
4000

Figure 4 B

```
         10         20         30         40         50         60         70
GAATTCCAATCCCACCAAAACCTGAACCTAGCAGTTCAGTTGCTCCTCTCAGAGACGAATCGGGTATTCA

         80         90         10        110        120        130        140
ACACCCTCATACCAACTACTACGTCGTGTATAACGGACCTCATGCCGGTATATACGATGACTGGGGTTGT

        150        160        170        180        190        200        210
ACAAAGGCAGCAACAAACGGTGTTCCCGGAGTTGCGCATAAGAAGTTTGCCACTATTACAGAGGCAAGAG

        220        230        240        250        260        270        280
CAGCAGCTGACGCGTATACAACAAGTCAGCAAACAGATAGGTTGAACTTCATCCCCAAAGGAGAAGCTCA

        290        300        310        320        330        340        350
ACTCAAGCCCAAGAGCTTTGCGAAGGCCCTAACAAGCCCACCAAAGCAAAAAGCCCACTGCTCACGCTAG

        360        370        380        390        400        410        420
GAACCAAAAGGCCCAGCAGTGATCCAGCCCCAAAAGAGATCTCCTTTGCCCCGGAGATTACAATGGACGA

        430        440        450        460        470        480        490
TTTCCTCTATCTTTACGATCTAGGAAGGAAGTTCGAAGGTGAAGGTGACGACACTATGTTCACCACTGAT

        500        510        520        530        540        550        560
AATGAGAAGGTTAGCCTCTTCAATTTCAGAAAGAATGCTGACCCACAGATGGTTAGAGAGGCCTACGCAG

        570        580        590        600        610        620        630
CAGGTCTCATCAAGACGATCTACCCGAGTAACAATCTCCAGGAGATCAAATACCTTCCCAAGAAGGTTAA

        640        650        660        670        680        690        700
AGATGCAGTCAAAAGATTCAGGACTAATTGCATCAAGAACACAGAGAAAGACATATTTCTCAAGATCAGA
```

Figure 4 B (cont. 1)

```
       710        720        730        740        750        760        770
AGTACTATTCCAGTATGGACGATTCAAGGCTTGCTTCATAAACCAAGGCAAGTAATAGAGATTGGAGTCT

       780        790        800        810        820        830        840
CTAAAAAGGTAGTTCCTACTGAATCTAAGGCCATGCATGGAGTCTAAGATTCAAATCGAGGATCTAACAG

       850        860        870        880        890        900        910
AACTCGCCGTGAAGACTGGCGAACAGTTCATACAGAGTCTTTTACGACTCAATGACAAGAAGAAAATCTT

       920        930        940        950        960        970        980
CGTCAACATGGTGGAGCACGACACTCTGGTCTACTCCAAAAATGTCAAAGATACAGTCTCAGAAGACCAA

       990       1000       1010       1020       1030       1040       1050
AGGGCTATTGAGACTTTTCAACAAAGGATAATTTCGGGAAACCTCCTCGGATTCCATTGCCCAGCTATCT

      1060       1070       1080       1090       1100       1110       1120
GTCACTTCATCGAAAGGACAGTAGAAAAGGAAGGTGGCTCCTACAAATGCCATCATTGCGATAAAGGAAA

      1130       1140       1150       1160       1170       1180       1190
GGCTATCATTCAAGATGCCTCTGCCGACAGTGGTCCCAAAGATGGACCCCCACCCACGAGGAGCATCGTG

      1200       1210       1220       1230       1240       1250       1260
GAAAAAGAAGACGTTCCAACCACGTCTTCAAAGCAAGTGGATTGATGTGACATCTCCACTGACGTAAGGG

      1270       1280       1290       1300       1310       1320       1330
ATGACGCACAATCCCACTATCCTTCGCAAGACCCTTCCTCTATATAAGGAAGTTCATTTCATTTGGAGAG

      1340       1350       1360       1370       1380
GACACGCTGAAATCACCAGTCTCTCTCTATAAATCTATCTCTCTCTCTATAACCATGG
```

Figure 5

```
         10        20        30        40        50
ATGACCATGATTACGAATTCGAGCTCGGTACCCGGGGATCCTCTAGAGTC
 M  T  M  I  T  N  S  S  S  V  P  G  D  P  L  E  S

         60        70    ↓   80        90       100
GACCTGCAGGGGGGGGGGGGGGGCTAAATTTGAACTCCAGCCTCCTCCTT
  T  C  R  G  G  G  G  A  K  F  E  L  Q  P  P  P

        110       120       130       140       150
ATCCCATGGATGCTTTGGAGCCTCATATGAGTAGTAGAACGTTTGAATTC
Y  P  M  D  A  L  E  P  H  M  S  S  R  T  F  E  F

        160       170       180       190       200
CACTGGGGGAAGCATCACAGGGCTTATGTCGACAATTTAAACAAGCAAAT
 H  W  G  K  H  H  R  A  Y  V  D  N  L  N  K  Q  I

        210       220       230       240       250
AGACGGAACAGAACTAGATGGAAAGACACTAGAAGACATAATACTTGTTA
  D  G  T  E  L  D  G  K  T  L  E  D  I  I  L  V

        260       270       280       290       300
CGTATAACAAAGGTGCTCCCCTCCCAGCATTCAACAATGCTGCTCAGGCC
T  Y  N  K  G  A  P  L  P  A  F  N  N  A  A  Q  A

        310       320       330       340       350
TGGAATCATCAGTTTTTCTGGGAATCAATGAAGCCCAACGGAGGAGGAGA
 W  N  H  Q  F  F  W  E  S  M  K  P  N  G  G  G  E

        360       370       380       390       400
GCCATCTGGTGAATTACTAGAACTAATCAACAGAGACTTTGGTTCCTATG
  P  S  G  E  L  L  E  L  I  N  R  D  F  G  S  Y

        410       420       430       440       450
ATGCATTTGTTAAAGAATTTAAGGCAGCTGCGGCAACACAATTTGGCTCT
D  A  F  V  K  E  F  K  A  A  A  A  T  Q  F  G  S

        460       470       480       490       500
GGTTGGGCCTGGCTCGCATACAAACCTGAAGAGAAAAAGCTTGCCTTGGT
 G  W  A  W  L  A  Y  K  P  E  E  K  K  L  A  L  V

        510       520       530       540       550
GAAAACTCCCAACGCTGAAAATCCTCTTGTTTTGGGTTACACACCGCTCC
  K  T  P  N  A  E  N  P  L  V  L  G  Y  T  P  L

        560       570       580       590       600
TCACCATAGACGTTTGGGAGCATGCTTACTATCTGGACTTTCAGAACCGG
L  T  I  D  V  W  E  H  A  Y  Y  L  D  F  Q  N  R

        610       620       630       640       650
CGGCCTGACTACATATCTATCTTTATGGAGAAGCTCGTGTCGTGGGAAGC
 R  P  D  Y  I  S  I  F  M  E  K  L  V  S  W  E  A
```

Figure 5 (continued)

```
       660       670       680       690       700
AGTCAGTTCTAGGCTTAAAGCAGCAACAGCTTGAGCTGCTTAGCGAGAAG
  V  S  S  R  L  K  A  A  T  A  .

       710       720       730       740       750
ACAGAAAGGAGGAAGAGGCAAATCTAGCAGGCACGAGAGTAAATATTTGA

       760       770       780       790       800
GACAGAATGATTTTTGTTAAAGAGACACTATTTTCAATCCTGCTATCCTT

       810       820       830       840       850
CTTTCTCAGTTGAGAATTTTAGATGTCTTATTATGTGCACTTTACTAGAG

       860       870       880       890       900
AGTCAAGTGATGCTCTGTATTTGGAGGATAGTGTTATTTCTGTTCTTTAG

       910       920       930       940       950
CAGCTGTTAATGGCAGGGAAAAATAATTCAAGTTGAGGTGTGGGACAACA

       960       970       980       990      1000
ATGTAAGGACGTGAATAAACAAATCTATTGCACTTTGGTGCCCTAATTTT

      1010      1020      1030
AGAATTAGAATGAAAAAAAACCCCCCCCCCCCCC
```

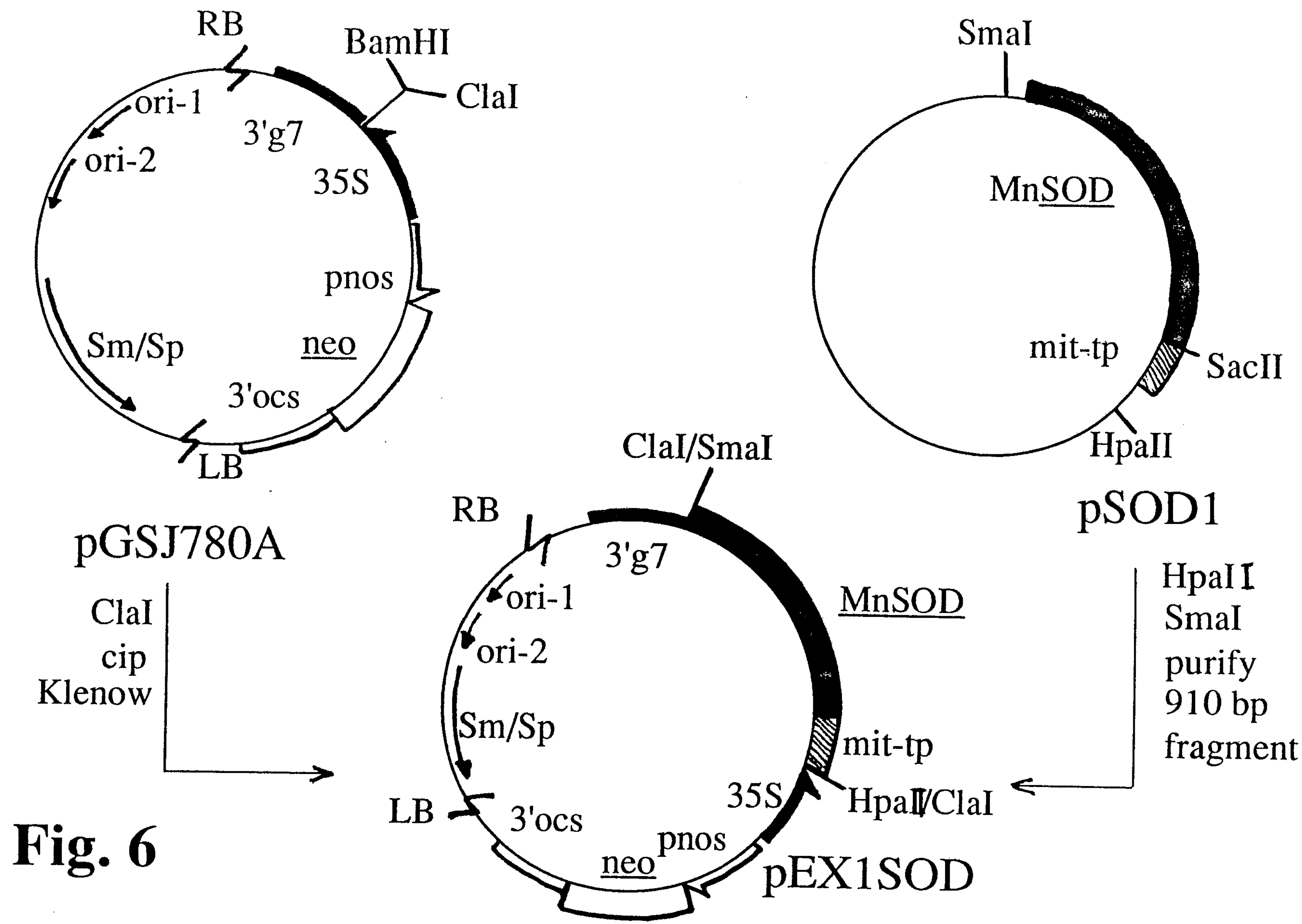
RB
BamHI
ClaI
ori-1
3'g7
ori-2
35S
pnos
Sm/Sp
neo
3'ocs
LB
pGSJ780A
ClaI
cip
Klenow
SmaI
MnSOD
mit-tp
SacII
HpaII
pSOD1
HpaII
SmaI
purify
910 bp
fragment
ClaI/SmaI
RB
3'g7
ori-1
MnSOD
ori-2
Sm/Sp
mit-tp
35S
HpaII/ClaI
LB
3'ocs
pnos
neo
pEX1SOD

Fig. 6

Fig. 7

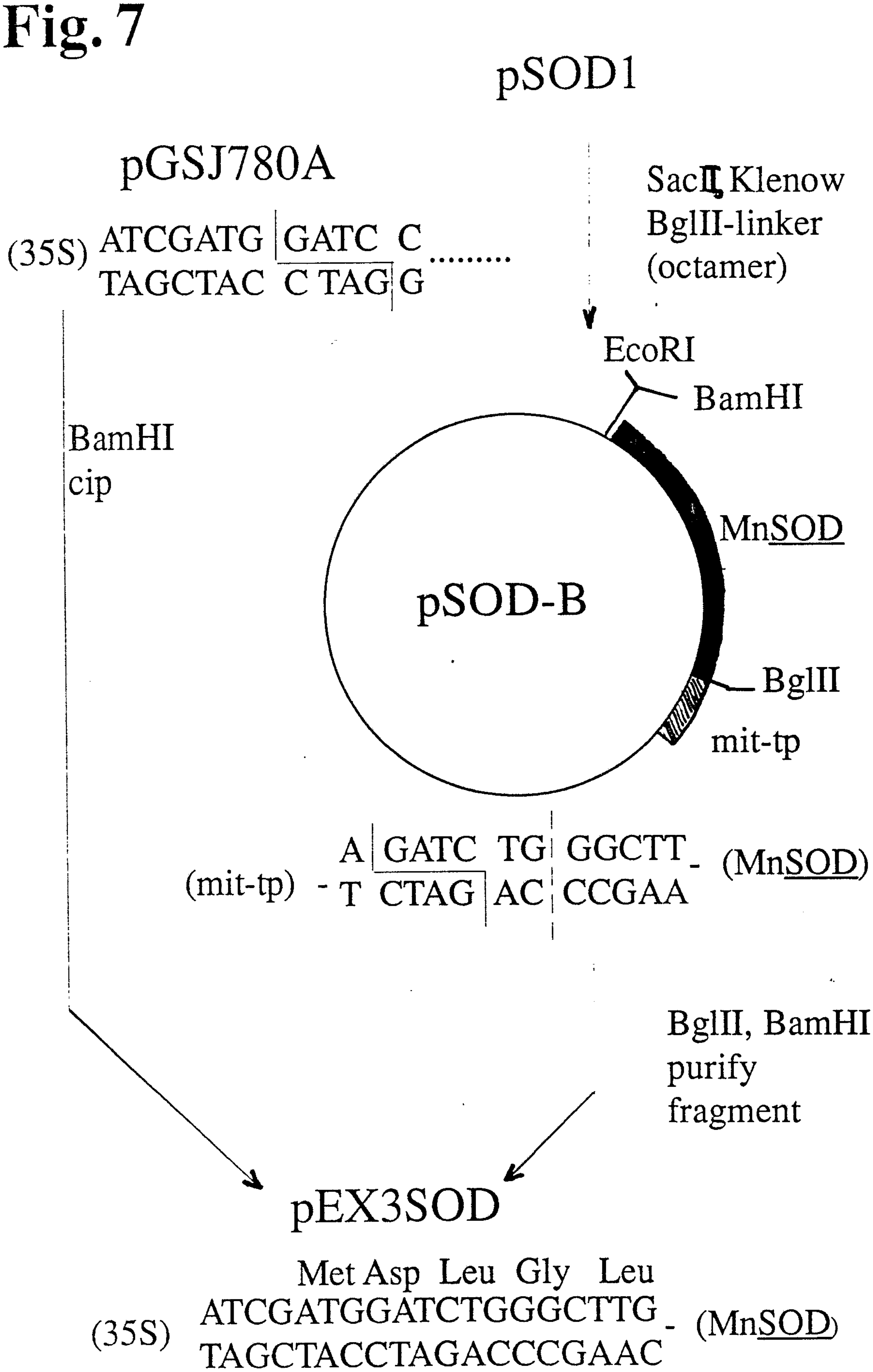
pSOD1
pGSJ780A
SacII, Klenow
BglII-linker
(octamer)
(35S) ATCGATG GATC C
TAGCTAC C TAG G .........
EcoRI
BamHI
BamHI
cip
MnSOD
pSOD-B
BglII
mit-tp
A GATC TG GGCTT
(mit-tp) - T CTAG AC CCGAA - (MnSOD)
BglII, BamHI
purify
fragment
pEX3SOD
Met Asp Leu Gly Leu
ATCGATGGATCTGGGCTTG
(35S) TAGCTACCTAGACCCGAAC - (MnSOD)

# Fig. 7 (cont.)

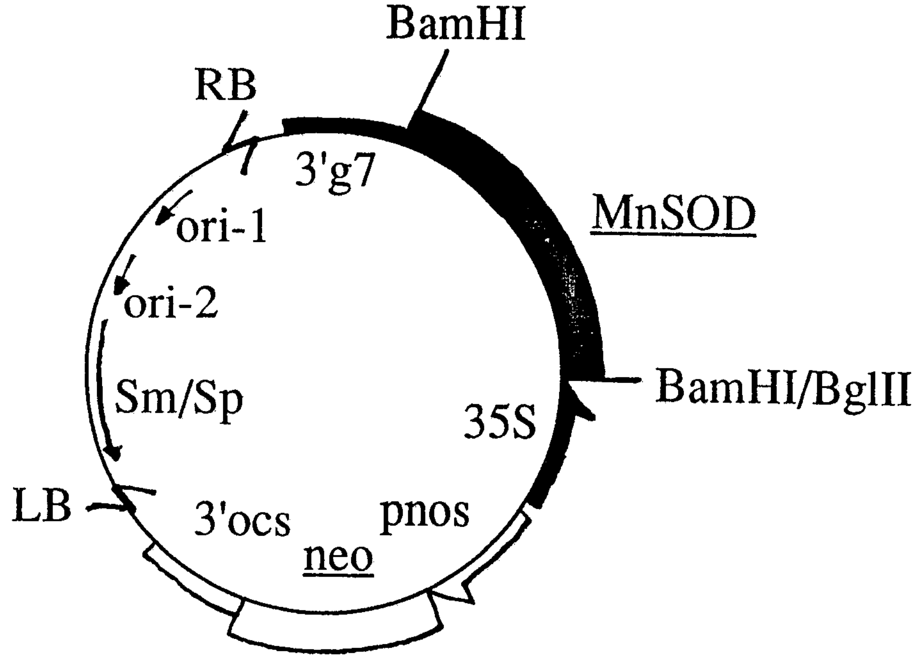


pEX3SOD

# Fig. 8

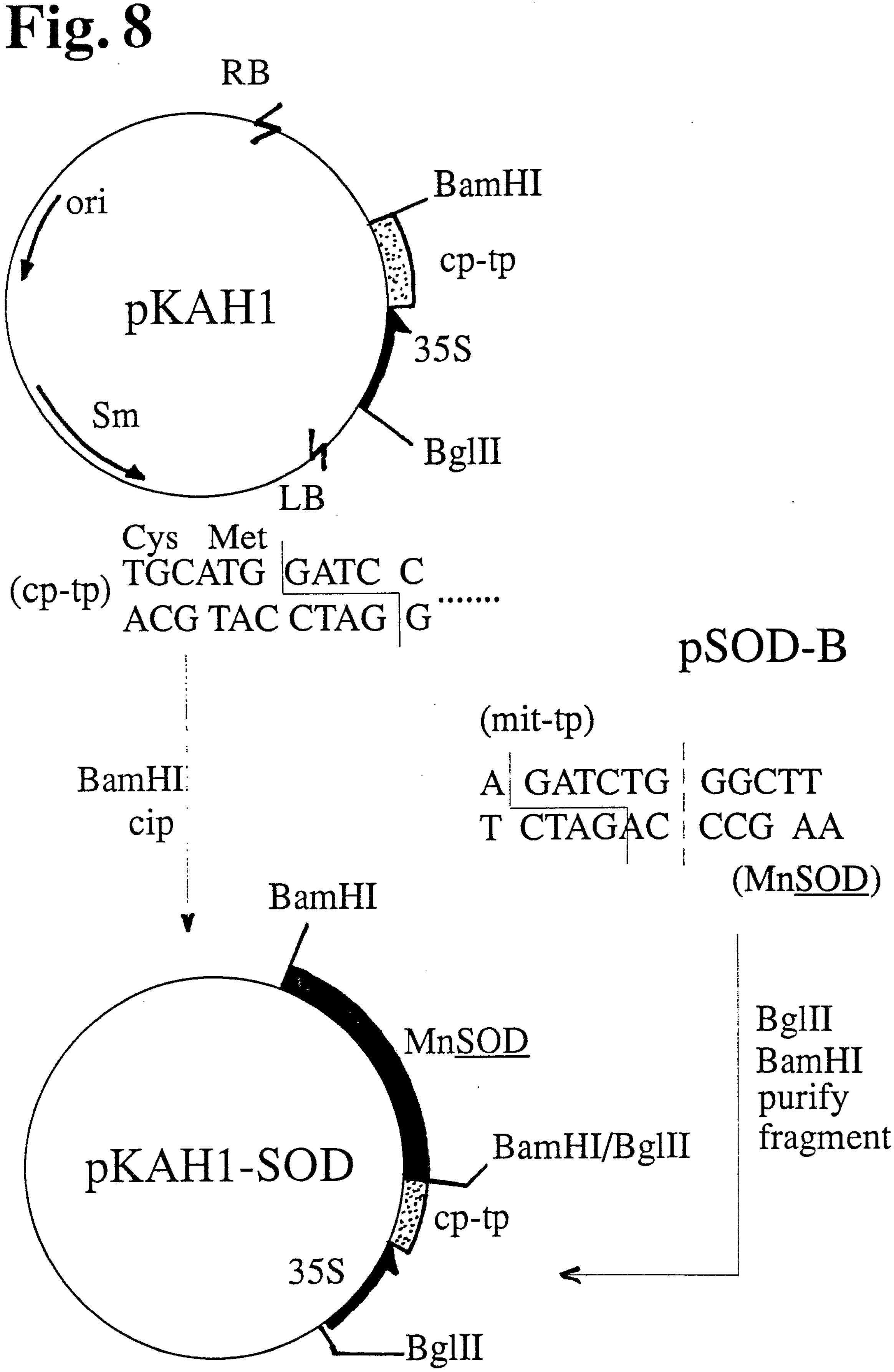

RB
ori
BamHI
cp-tp
pKAH1
35S
Sm
BglII
LB
Cys Met
TGCATG GATC C
(cp-tp)
ACG TAC CTAG G .......
pSOD-B
(mit-tp)
BamHI
cip
A GATCTG GGCTT
T CTAGAC CCG AA
(MnSOD)
BamHI
MnSOD
BglII
BamHI
purify
fragment
pKAH1-SOD
BamHI/BglII
cp-tp
35S
BglII

# Fig. 8 (cont.)

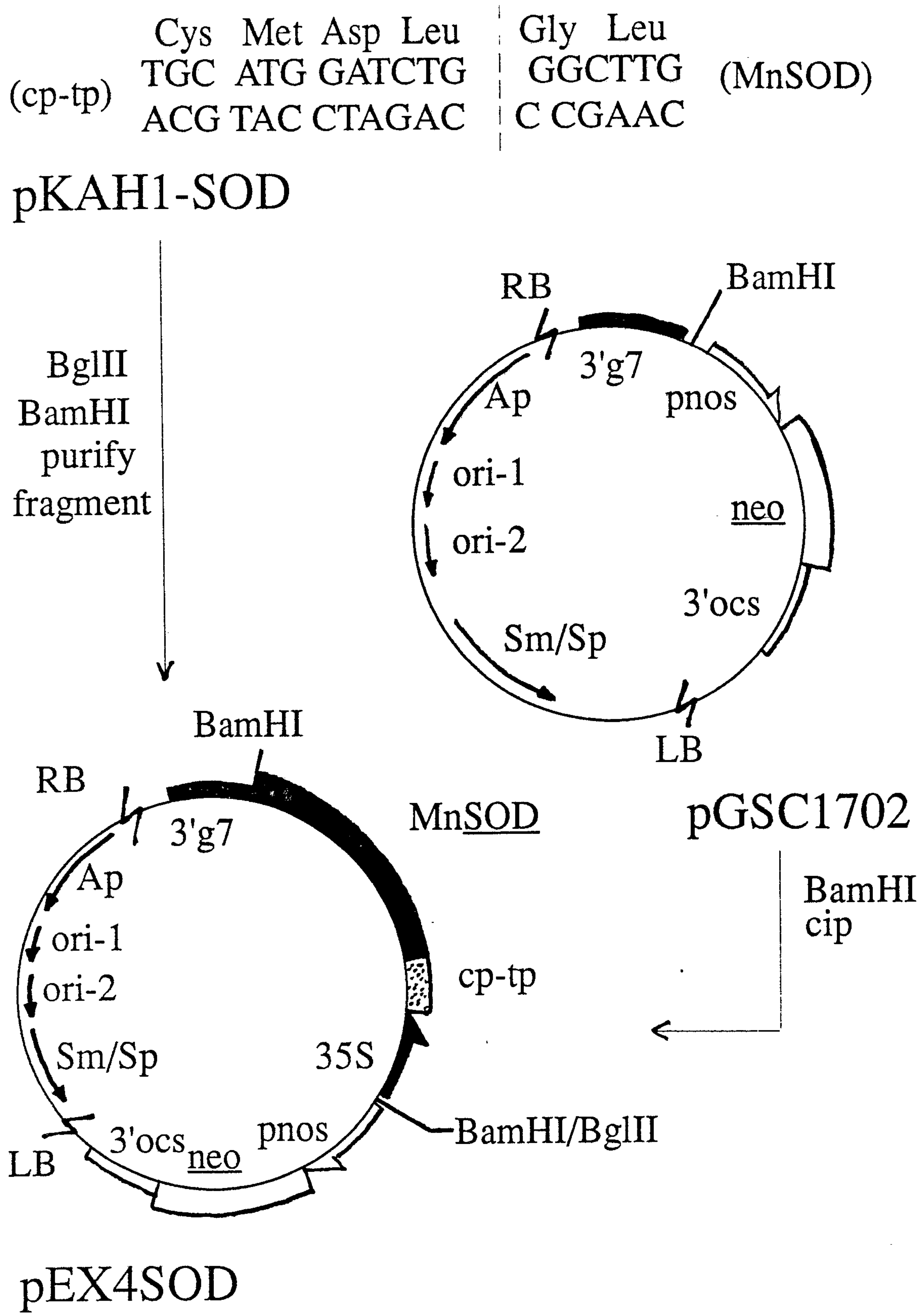
Cys Met Asp Leu
TGC ATG GATCTG
ACG TAC CTAGAC
(cp-tp)
Gly Leu
GGCTTG
C CGAAC
(MnSOD)
pKAH1-SOD
BglII
BamHI
purify
fragment
RB
BamHI
3'g7
Ap
pnos
ori-1
neo
ori-2
3'ocs
Sm/Sp
LB
pGSC1702
BamHI
cip
BamHI
RB
3'g7
MnSOD
Ap
ori-1
ori-2
cp-tp
Sm/Sp
35S
pnos
3'ocs
neo
LB
BamHI/BglII
pEX4SOD

# Fig. 9

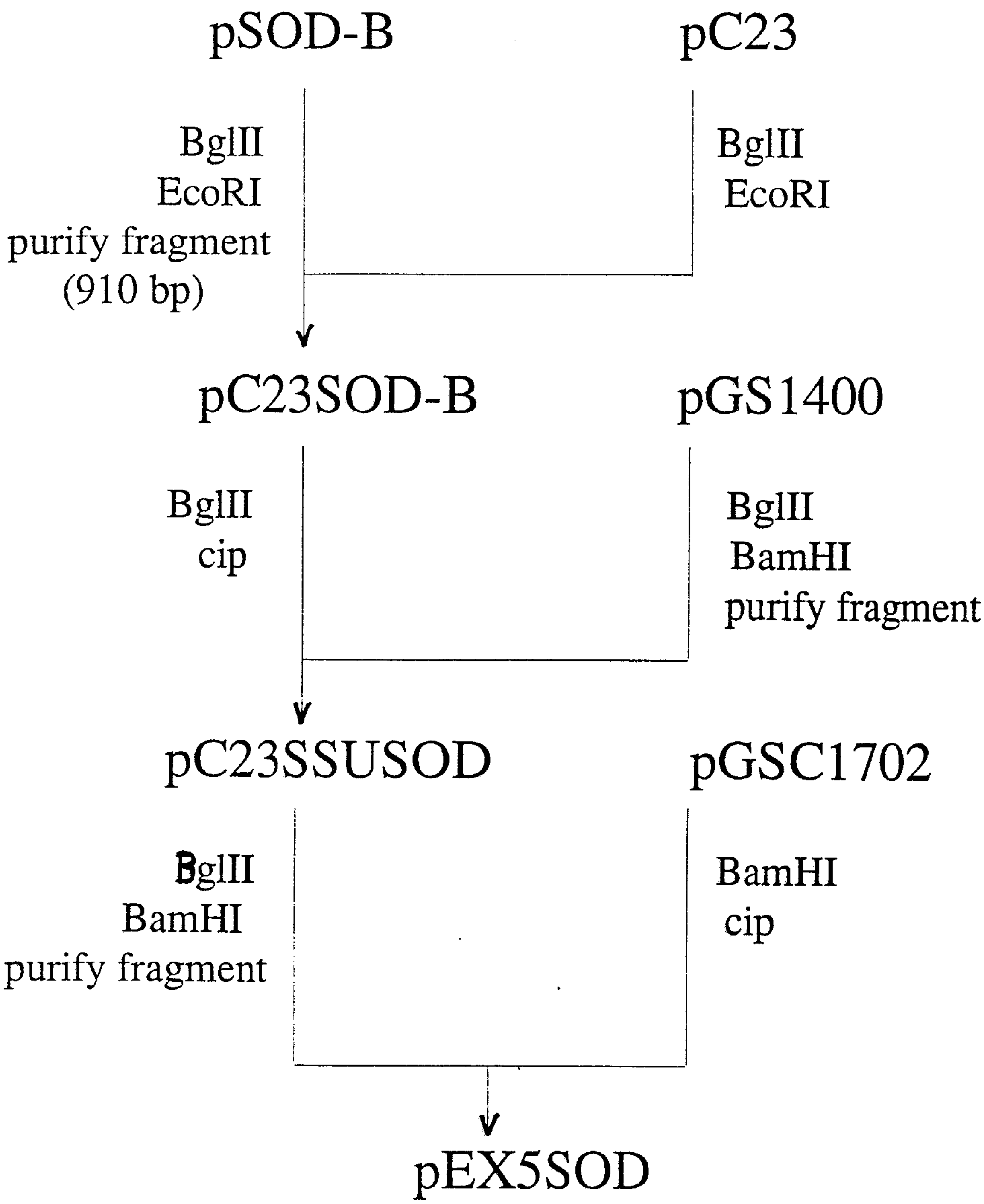
pSOD-B
pC23
BglII
EcoRI
purify fragment
(910 bp)
BglII
EcoRI
pC23SOD-B
pGS1400
BglII
cip
BglII
BamHI
purify fragment
pC23SSUSOD
pGSC1702
BglII
BamHI
purify fragment
BamHI
cip
pEX5SOD

# Fig. 9 (cont.)

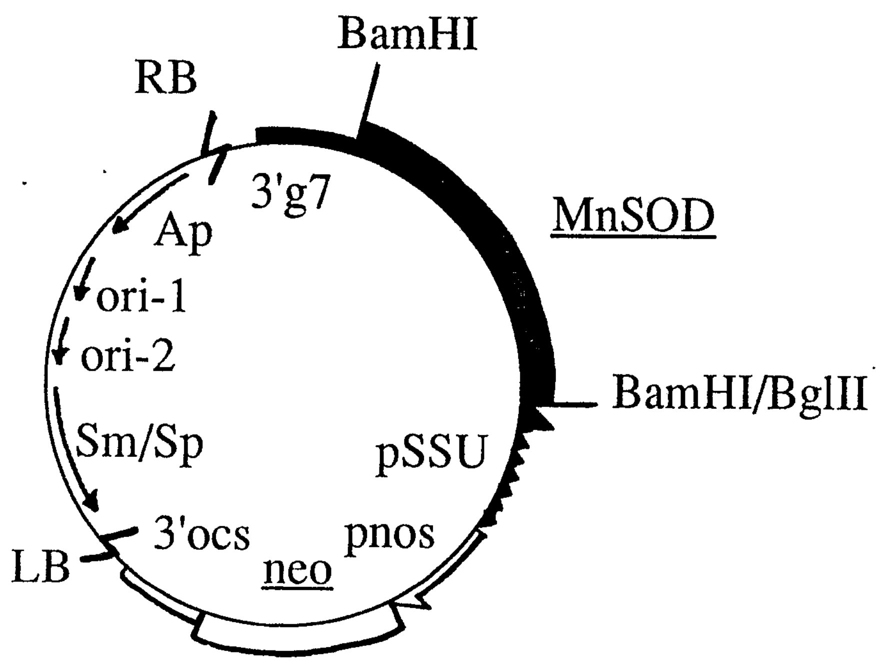


**pEX5SOD**

# Fig. 10

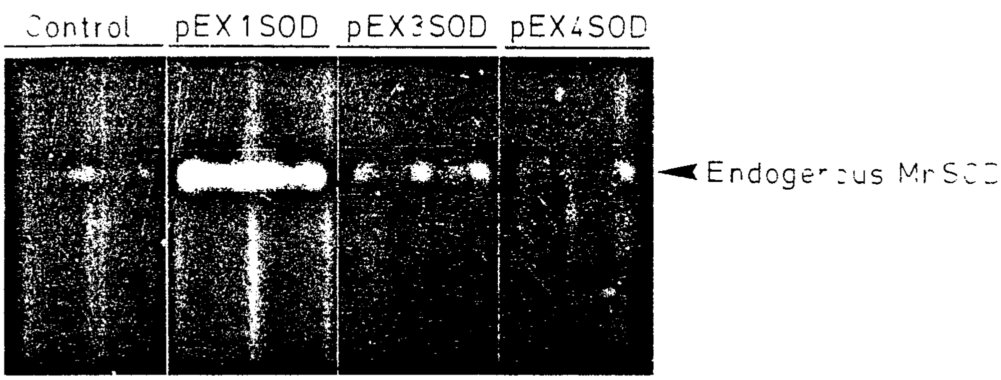

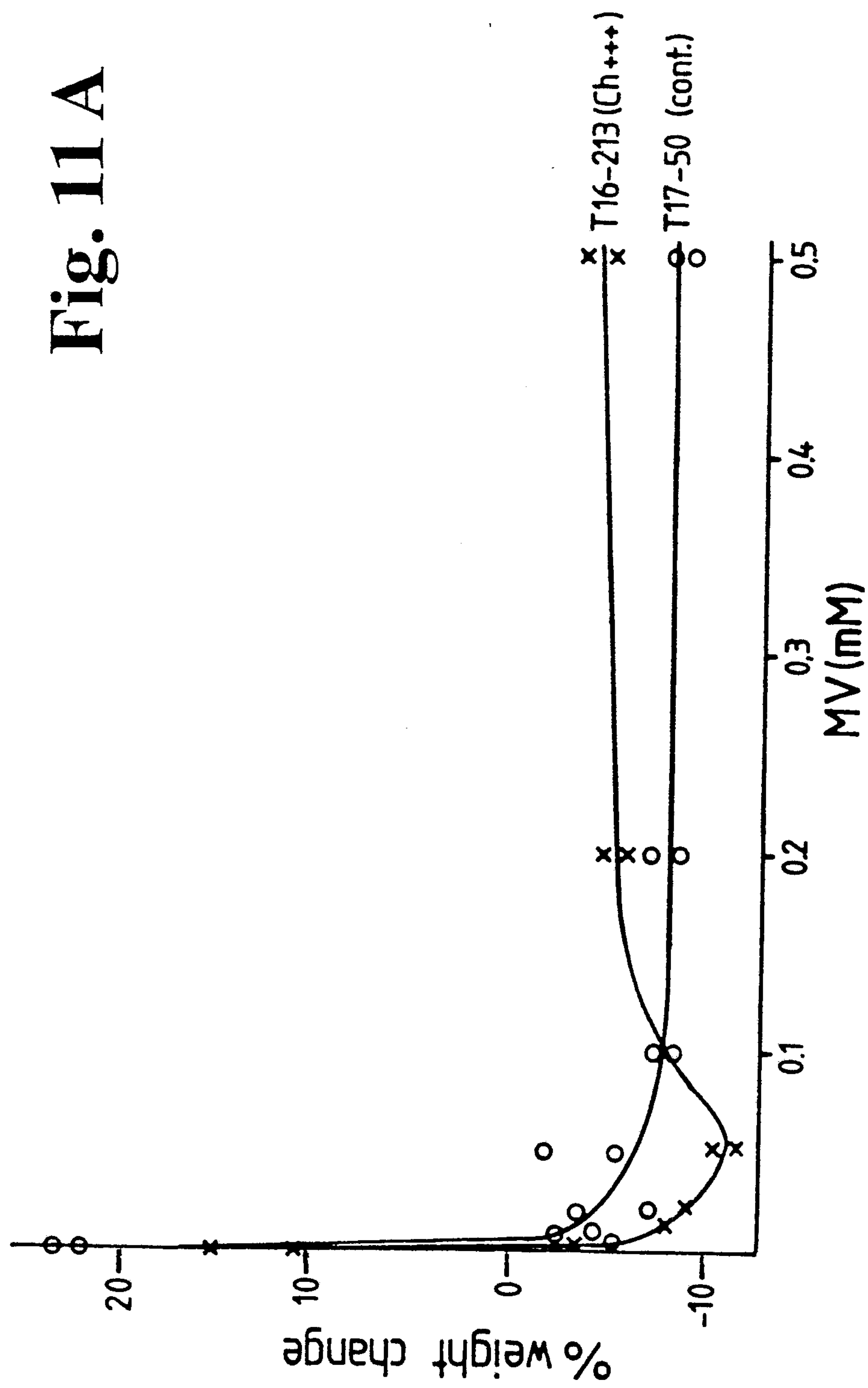
% weight change
20
10
0
-10
MV (mM)
0.1
0.2
0.3
0.4
0.5
T16-213 (Ch+++)
T17-50 (cont.)

Fig. 11 A

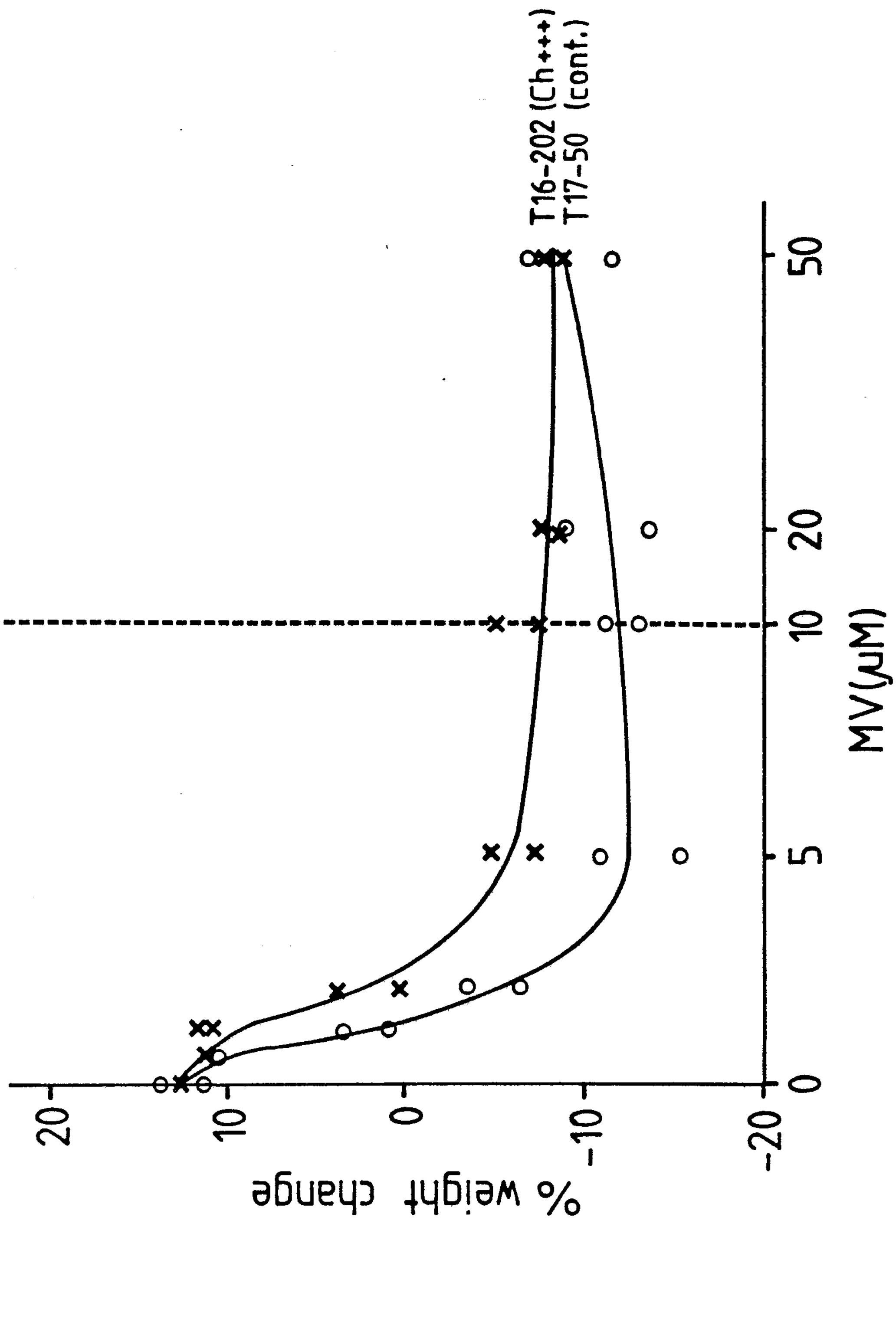

% weight change
20
10
0
-10
-20
0
5
10
20
50
MV(µM)
T16-202 (Ch+++)
T17-50 (cont.)


Fig. 11 B

Fig. 12 A

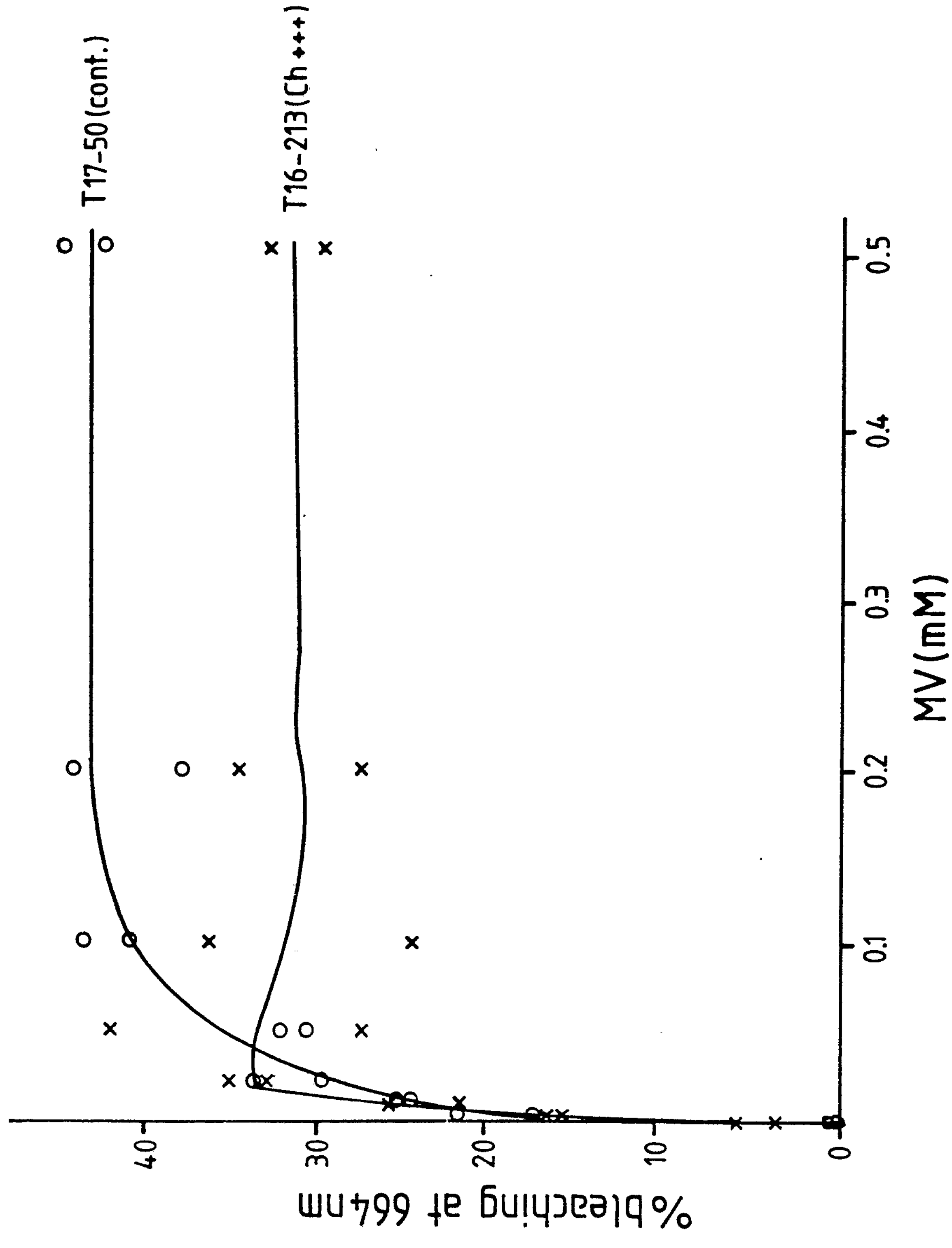
T17-50 (cont.)
T16-213 (Ch +++)
% bleaching at 664 nm
0
10
20
30
40
MV (mM)
0.1
0.2
0.3
0.4
0.5

# Fig. 12 B

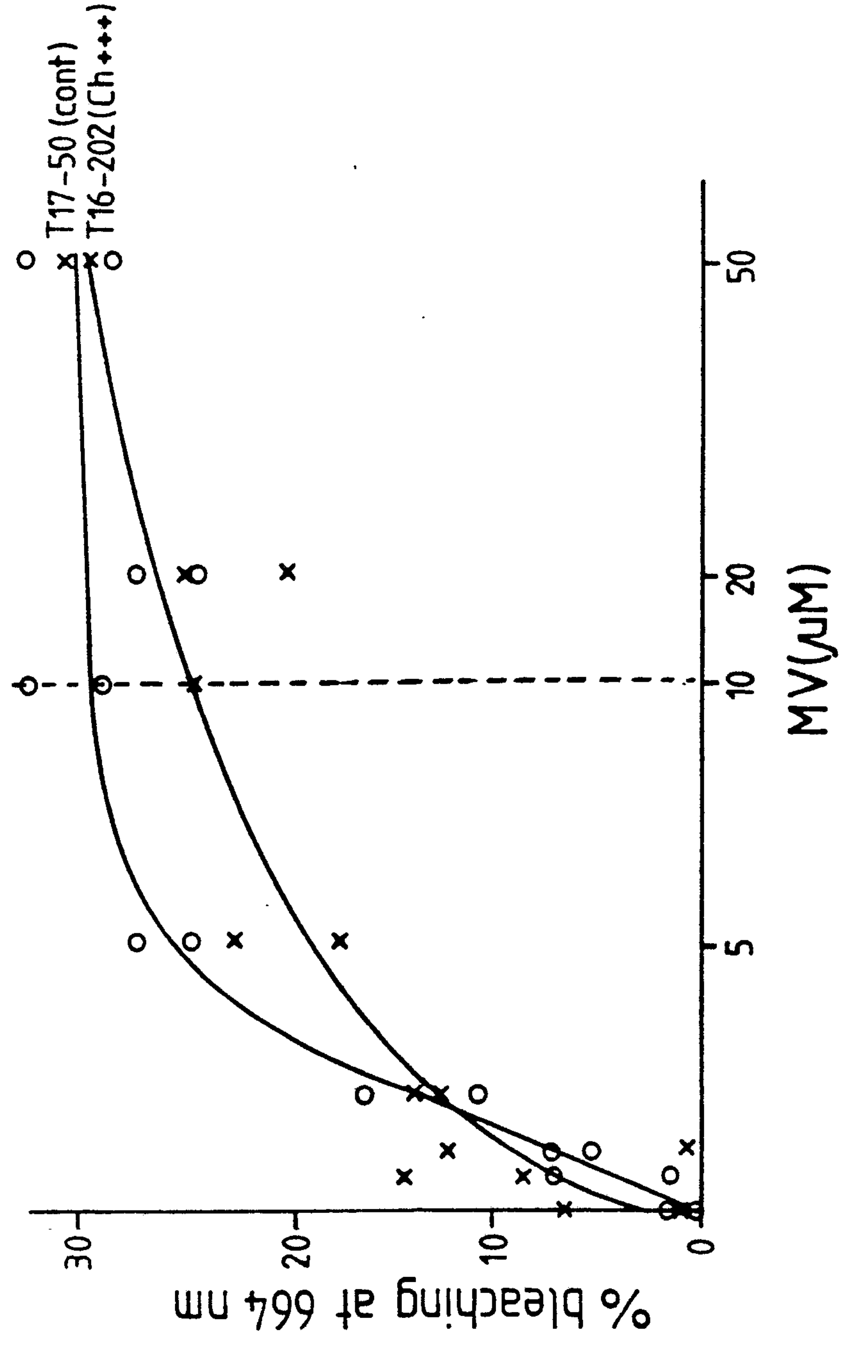
T17-50 (cont)
T16-202 (Ch+++)
% bleaching at 664 nm
0
10
20
30
5
10
20
50
MV(μM)

# Fig. 13

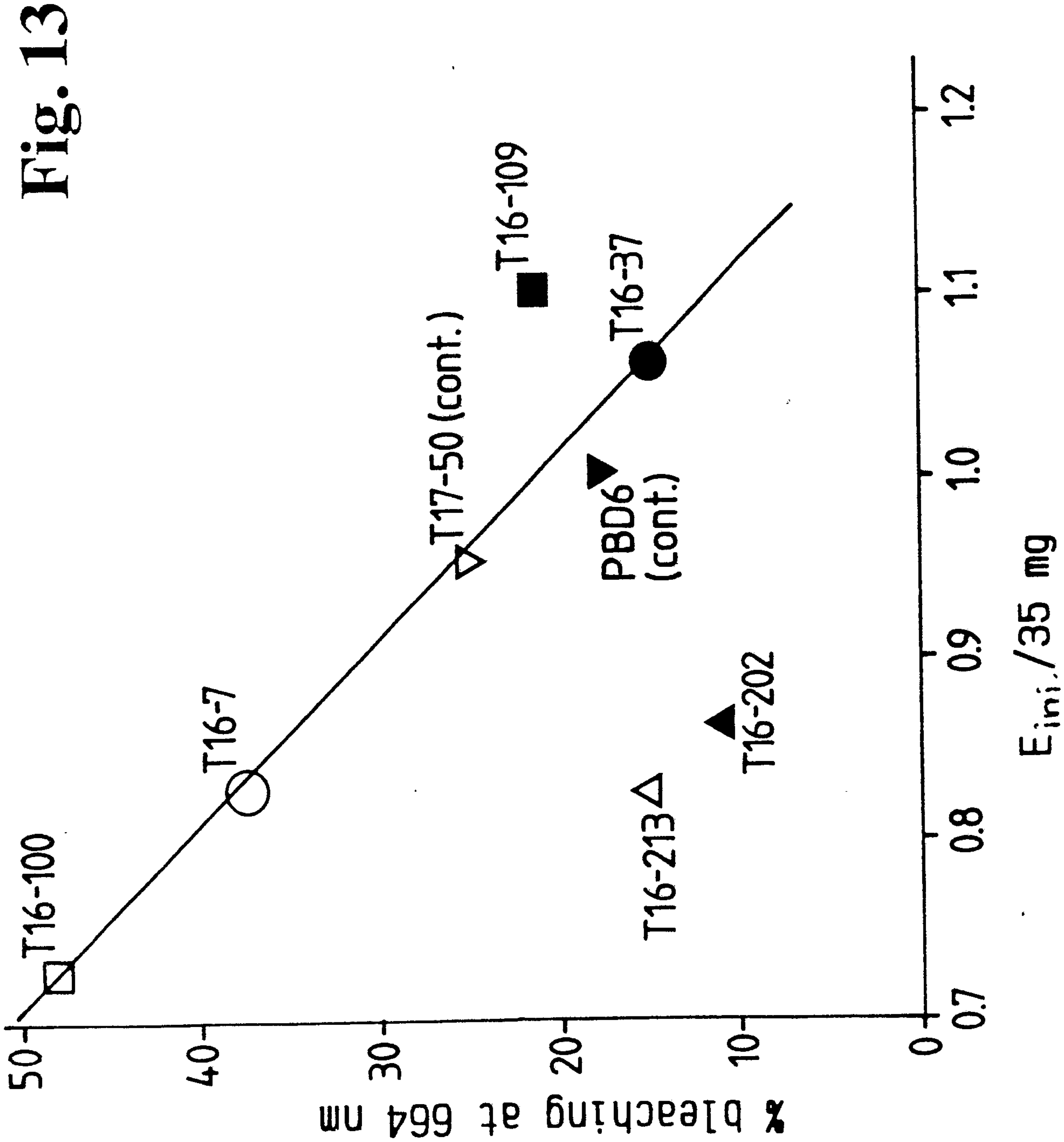
% bleaching at 664 nm
50
40
30
20
10
0
0.7
0.8
0.9
1.0
1.1
1.2
$E_{ini.}$/35 mg
T16-100
T16-7
T17-50 (cont.)
T16-109
T16-37
PBD6 (cont.)
T16-202
T16-213

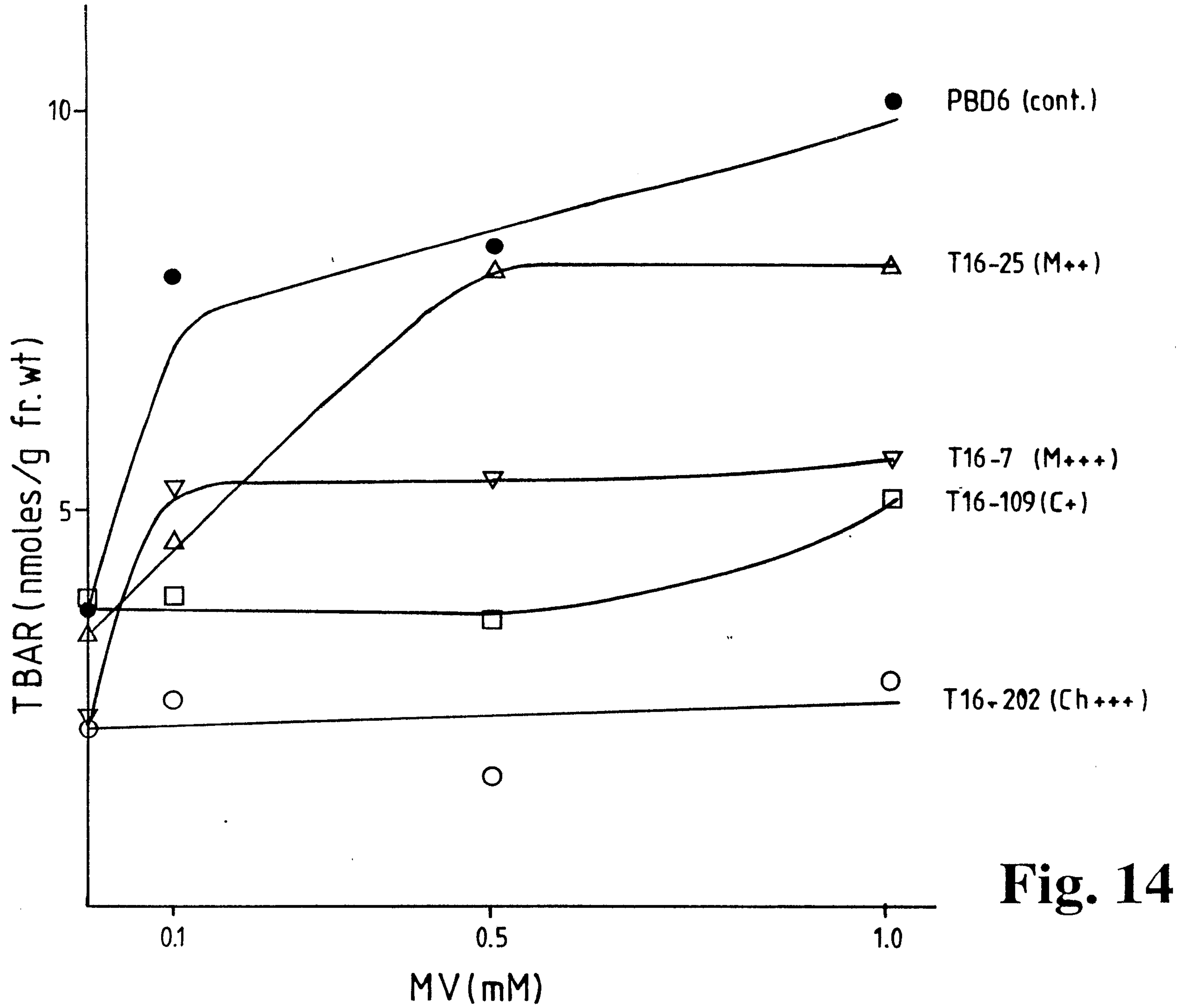
PBD6 (cont.)
T16-25 (M++)
T16-7 (M+++)
T16-109 (C+)
T16-202 (Ch+++)
TBAR (nmoles/g fr.wt)
10
5
0.1
0.5
1.0
MV (mM)

Fig. 14 A

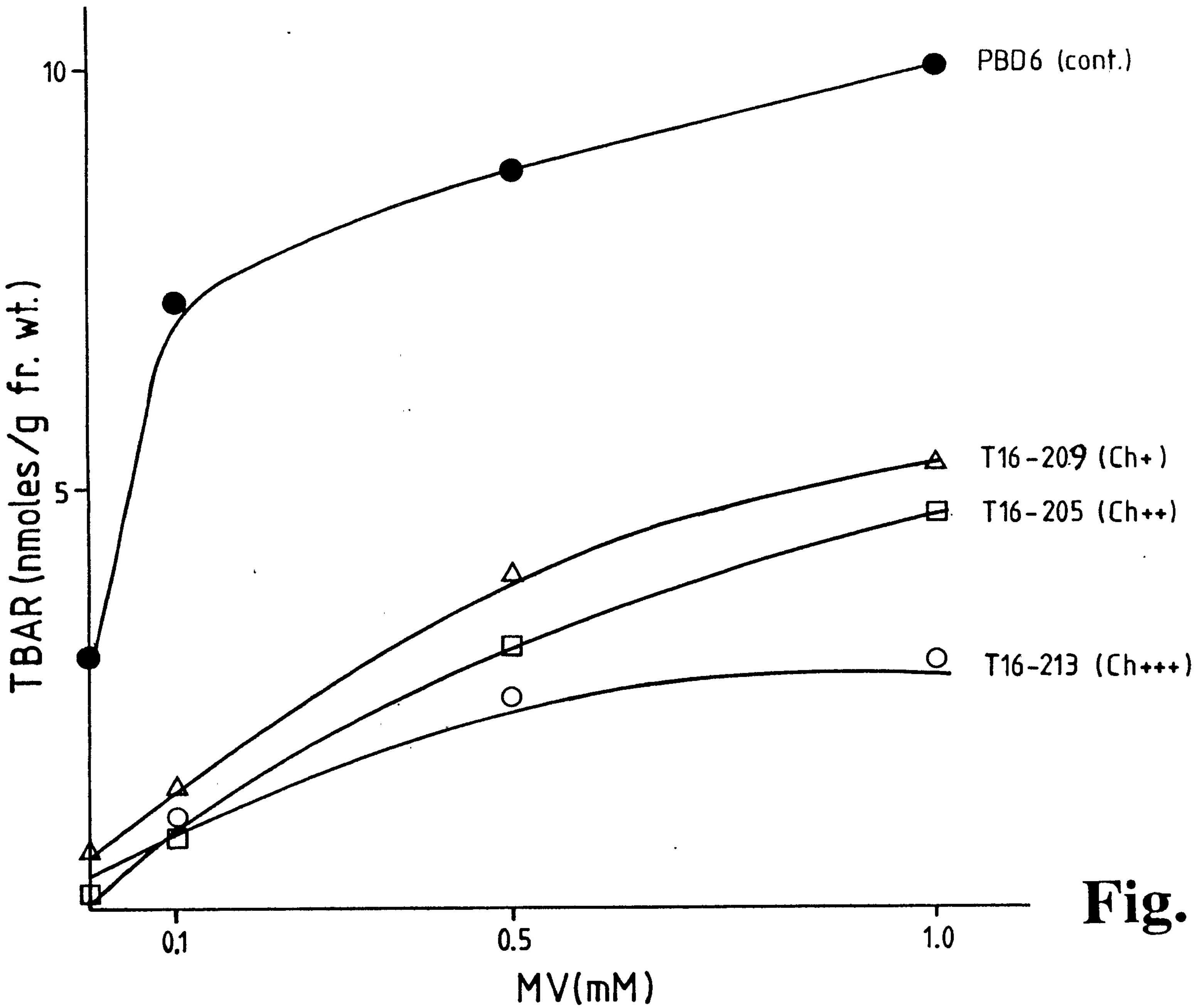
10
5
TBAR (nmoles/g fr. wt.)
PBD6 (cont.)
T16-209 (Ch+)
T16-205 (Ch++)
T16-213 (Ch+++)
0.1
0.5
1.0
MV(mM)

Fig. 14 B